# EUROPEAN PATENT APPLICATION

(11) **EP 3 708 093 A1**
(43) Date of publication of application: **16.09.2020**
(21) Application number: 19184477.8
(22) Date of filing: 04.07.2019
(51) Int. Cl.: A61B 17/28

(54) **AMBIDEXTROUS LOCKING CLAMP SYSTEM**

(30) Priority: 14.03.2019 US 201916353010
(71) Applicant: Biolchini Jr., Robert F., Jackson, WY 83001 (US)
(72) Inventor: Biolchini Jr., Robert F., Jackson, WY 83001 (US)
(74) Representative: KLIMENT & HENHAPEL

(57) **Abstract**

An ambidextrous locking clamp system (10, 60, 100, 150, 180, 200, 220, 250, 270, 290, 310, 400, 500, 550, 600) for providing a user the ability to use the right or left hand to engage and disengage a ratcheting means on the clamp. The clamp includes hingedly connected first and second elongated members each with a finger engaging member (16, 66, 156, 186, 206, 226, 256, 272, 296, 316, 414, 512, 562, 614), a working head (13, 63, 103, 153, 183, 203, 223, 253, 273, 293, 313, 404, 432, 504, 522, 554, 572, 604, 624, 660, 676), and a latching member (80, 130, 210, 260, 300, 340, 416, 514, 564) featuring ratcheting teeth. The teeth are engaged by moving the finger engaging members (16, 18; 66, 68; 156, 158; 186, 188;, 206, 208; 226, 228; 256, 258; 272, 278;, 296, 298; 316, 318; 414, 440; 512, 528; 562, 578; 614, 632) toward each other in an engaging motion, and are disengaged by sliding them in a disengaging motion perpendicular to the engaging motion with opposing force applied to the finger engaging members (16, 18; 66, 68; 156, 158; 186, 188;, 206, 208; 226, 228; 256, 258; 272, 278;, 296, 298; 316, 318; 414, 440; 512, 528; 562, 578; 614, 632). One of the elongated members includes a planar portion that is rotatably received in a slot (674) defined in a female portion of the other elongated member. The planar portion can include a wedge that contacts a side of the female portion (672) that defines the slot (674) when the clamp is in a closed position.

## Description

### CROSS REFERENCE TO RELATED APPLICATION

This application claims priority of U.S. continuation-in-part application No. 16/353.010 based upon co-pending U.S. patent application No. 15/070,272 filed on March 15,2016, which is a continuation-in-part application of U.S. patent application No. 13/272,676 filed on October 13,2011 now patent U.S. 9,427,245 issued on August 30, 2016, which is a continuation-in-part application of U.S. patent application No. 11/733,280 filed on April 10, 2007 now patent U.S. 8,070,771 issued on December 6, 2011, which is a continuation-in-part application of U.S. application No. 10/909,623 filed on August 2, 2004 now patent US 7,758,609 issued on July 20, 2010.

### BACKGROUND

### TECHNICAL FIELD

The present technology relates to an ambidextrous locking clamp system for use in connection with clamping instruments, such as surgical clamps, forceps, or hemostats. The ambidextrous locking clamp system has particular utility in connection with manipulating a hand tool to engage latching members by moving flexible members toward each other which, and to disengage the latching members by sliding the latching members in a motion perpendicular to the engaging motion thereby flexing the flexible members away from each other when an opposing force is applied to the flexible members. The opposing force is produced by pushing with a thumb of an operating hand of a user on one of the flexible members and pulling with fingers of the operating hand on the other flexible member thereby slidably separating the latching members.

### BACKGROUND DESCRIPTION

Ambidextrous locking clamps, forceps or hemostats are desirable for allowing a right or left-handed user to use a single hand operated clamp, forceps or hemostat device. These hand operated devices have been manufactured in the past for either a right hand or left hand user. This manufacturing process has some disadvantages in that the manufacturer would have to make a decision as to how many right handed and left handed devices to fabricate. In most cases, the decision is made to manufacture more right-handed devices than left handed devices. Therefore, it is well known that it is very difficult for a left-handed user to operate a right-handed device.

Hand operated locking clamps, forceps and hemostats are known in the art. These devices include a pair of elongated members joined by a hinge. The hinge is usually a hinge pin extending through both elongated members. One end of the elongated members features a working head, usually a griping jaw or cutting edges. The other end of the elongated members feature a finger engaging loop, with a set of ratchet teeth extending out therefrom towards the ratchet teeth of the finger loop of the second elongated member. The ratchet teeth are orientated so that they engage each other when the finger loop ends are brought together. These devices are mainly used in the medical industry for a wide variety of uses, but they are also used in the fly fishing, model building, and electrical industries.

During operation of a standard right handed hand operated device, the user inserts his or her thumb into one loop, the middle finger in the opposite loop, and the index finger would rest on the top of the middle finger loop for support and control of the device. To engage the working head the user squeezes the thumb and middle finger together guided by the index finger. The device is locked in the close position by further squeezing the loops together until the ratchet teeth members engage each other. To release, the thumb pushes away from the palm of the hand and the middle finger pulls toward the palm of the hand. This motion makes the ratchet teeth members flex away from each other and disengage.

The difficulty lies when a left-handed user tries to operate a right-handed device. It is difficult for a left-handed user to pull with the thumb and push with the middle finger. This is not a natural hand motion.

While the above-described devices fulfill their respective, particular objectives and requirements, the aforementioned patents do not describe an ambidextrous hand operated device that allows the use of the device by a right or left handed user through the engaging of latching members by moving flexible members toward each other which, and the disengaging of the latching members by sliding the latching members in a motion perpendicular to the engaging motion thereby flexing the flexible members away from each other when an opposing force is applied to the flexible members. Wherein, the opposing force is produced by pushing with a thumb of an operating hand of a user on one of the flexible members and pulling with fingers of the operating hand on the other flexible member thereby slidably separating the latching members.

Therefore, a need exists for a new and improved ambidextrous locking clamp system that can be used for manipulating objects with a tool having removable and interchangeable components. In this regard, the present technology substantially fulfills this need. In this respect, the ambidextrous locking clamp system according to the present technology substantially departs from the conventional concepts and designs of the prior art, and in doing so provides an apparatus primarily developed for the purpose of manipulating a hand tool to engage latching members by moving flexible members toward each other which, and to disengage the latching members by sliding the latching members in a motion perpendicular to the engaging motion thereby flexing the flexible members away from each other when an opposing force is applied to the flexible members. The opposing force is produced by pushing with a thumb of an operating hand of a user on one of the flexible members and pulling with fingers of the operating hand on the other flexible member thereby slidably separating the latching members.

### BRIEF SUMMARY OF THE PRESENT TECHNOLOGY

In view of the foregoing disadvantages inherent in the known types of hand operated locking devices, the present technology provides an improved ambidextrous locking clamp system, and overcomes the above-mentioned disadvantages and drawbacks of the prior art. As such, the general purpose of the present technology, which will be described subsequently in greater detail, is to provide a new and improved ambidextrous locking clamp system and method which has all the advantages of the prior art mentioned heretofore and many novel features that result in a ambidextrous locking clamp system which is not anticipated, rendered obvious, suggested, or even implied by the prior art, either alone or in any combination thereof.

According to one aspect of the present technology, the present technology essentially includes an ambidextrous locking clamp system for allowing a right hand or left hand user to operate said clamp. The system can include a first member and a second member pivotably connected with each other. The first member can include at least one first elongated body, at least one first finger engaging member, and at least one first latching member. The first elongated body can include a planar portion, and at least one first working head located at an end of the first elongated body. The first latching member can include first member ratcheting teeth. The second member can include at least one second elongated body, at least one second finger engaging member, and at least one second latching member. The second elongated body can include a female portion defining a slot, and at least one second working head located at an end of the second elongated body. The slot can be configured to receive the planar portion of the first member. The second latching member can include at least one second member ratcheting teeth. The first and second members being in a pivotable relationship about a pivot point so that the first and second working heads move toward each other when the first and second finger engaging member are moved toward each other.

According to an aspect of the present technology, the present technology essentially includes an ambidextrous locking clamp system. The system can include a first member and a second member pivotably connected with each other. The first member can include at least one first elongated body, at least one first finger engaging member, and at least one first latching member. The first elongated body can include a planar portion, and at least one first working head located at an end of the first elongated body. The first latching member can include first member ratcheting teeth. The second member can include at least one second elongated body, at least one second finger engaging member, and at least one second latching member. The second elongated body can include a female portion defining a slot, and at least one second working head located at an end of the second elongated body. The slot can be configured to receive the planar portion of the first member. The second latching member can include second member ratcheting teeth. The first and second members being pivotably connected about a pivot point so that the first and second working heads move toward each other when the first and second finger engaging member are moved toward each other. The first and second member ratcheting teeth can be orientated to be engageable with each other by an engaging motion when the first and second finger engaging members are moved toward each other.

According to yet another aspect of the present technology, the present technology can include a method of using an ambidextrous locking clamp system. The method can include the steps of operating a first finger engaging member of a first member and a second finger engaging member of a second member by a user to move the first and second finger engaging members toward each other about a pivot point in an engaging motion. The pivot point can be configured to pivotably connect the first and second members to each other. Rotating a planar portion of the first member and a female portion of the second member about the pivot point during the engaging motion. The planar portion can be receivable in a slot defined in the female portion. Engaging ratcheting teeth of first and second members with each other by the engaging motion until the ratcheting teeth of the first and second members overlap one another in succession to a user desired tension when a working head of the first member and the second member are in a closed position. Disengaging the ratcheting teeth of the first and second members by a disengaging motion perpendicular to the engaging motion until the ratcheting teeth are slidably disengaged. The disengaging motion can be produced by moving the first and second members in opposite directions when an opposing force is applied to the first and second finger engaging members by pushing on at least one of the first and second finger engaging members and pulling on the other of the first and second finger engaging members thereby slidably separating the ratcheting teeth of the first and second members out of engagement

In some embodiments of the present technology, operating the first finger engaging member of the first member can be accomplished by utilizing at least a thumb or a finger of an operating hand of the user, while operating the second finger engaging member of the second member by at least a thumb or a finger of the operating hand of the user that is not used in operating the first finger engaging member.

In some embodiments of the present technology, the disengaging motion can be produced by moving the first and second members in opposite directions when an opposing force is applied to the first and second finger engaging members by pushing with the thumb of the operating hand on at least one of the first and second finger engaging members and pulling with the finger of the operating hand on the other of the first and second finger engaging members.

In some embodiments of the present technology, the planar portion can include at least one wedge configured to contact a side of said female portion that defines the slot when the first and second working heads are in a closed position.

In some embodiments of the present technology, the wedge can extend from the planar portion so that an exterior edge of the wedge is flush with an edge of the planar portion.

In some embodiments of the present technology, the wedge can include an exterior side and an interior tapered side. The wedge can be configured so that the interior tapered side enters the slot prior to the exterior side during the engaging motion.

In some embodiments of the present technology, the can be a pair of wedges each extending from opposite sides of the planar portion away from each other.

In some embodiments of the present technology, the wedge can be adjacent the first elongated body, with the pivot point being located between the wedge and the working head of the first member.

Some embodiments of the present technology, the planar portion can include at least one secondary wedge configured to contact a side of the female portion that defines the slot when the first and second working heads are in a closed position.

In some embodiments of the present technology, the secondary wedge can include an exterior side and an interior tapered side. The secondary wedge can be configured so that the interior tapered side of the secondary wedge enters the slot prior to the exterior side of the secondary wedge during the engaging motion.

In some embodiments of the present technology, the secondary wedge can be a pair of secondary wedges each extending from opposite sides of the planar portion away from each other.

In some embodiments of the present technology, the secondary wedge can be adjacent the working head of the first member, with the pivot point being located between the wedge and the secondary wedge.

In some embodiments of the present technology, the wedge and the secondary wedge can be located on opposite sides of a longitudinal axis of the first member.

Some embodiments of the present technology can include the first latching member received through a first member opening defined through the first elongated member, and the second latching member received through a second member opening defined through the second elongated member.

In some embodiments of the present technology, the first member opening can be a recess defined by a bent portion of the first elongated body, and the second member opening can be a recess defined by a bent portion of the second elongated body.

In some embodiments of the present technology, the first member opening can be a bore defined through the first elongated body, and the second member opening can be a bore defined through the second elongated body.

In some embodiments of the present technology, the first and second member ratcheting teeth can have a configuration for disengaging with each other by sliding the first and second member ratcheting teeth apart by a disengaging motion perpendicular to the engaging motion resulting in moving the first and second latching members away from each other when an opposing force is applied to the first and second finger engaging members.

There has thus been outlined, rather broadly, the more important features of the technology in order that the detailed description thereof that follows may be better understood and in order that the present contribution to the art may be better appreciated.

The technology may also include a variety of latching members, such as, but not limited to, rigid latching members, flexible latching members, flexible armed latching members, and ratcheting heads. There are, of course, additional features of the technology that will be described hereinafter and which will form the subject matter of the claims attached.

Numerous embodiments, features and advantages of the present technology will be readily apparent to those of ordinary skill in the art upon a reading of the following detailed description of presently preferred, but nonetheless illustrative, embodiments of the present technology when taken in conjunction with the accompanying drawings. In this respect, before explaining the current embodiment of the technology in detail, it is to be understood that the technology is not limited in its application to the details of construction and to the arrangements of the components set forth in the following description or illustrated in the drawings. The technology is capable of other embodiments and of being practiced and carried out in various ways. Also, it is to be understood that the phraseology and terminology employed herein are for the purpose of descriptions and should not be regarded as limiting.

As such, those skilled in the art will appreciate that the conception, upon which this disclosure is based, may readily be utilized as a basis for the designing of other structures, methods and systems for carrying out the several purposes of the present technology. It is important, therefore, that the claims be regarded as including such equivalent constructions insofar as they do not depart from the spirit and scope of the present technology.

It is therefore an embodiment of the present technology to provide a new and improved ambidextrous locking clamp system that has all of the advantages of the prior art locking clamps and none of the disadvantages.

It is another embodiment of the present technology to provide a new and improved ambidextrous locking clamp system that may be easily and efficiently manufactured and marketed.

An even further embodiment of the present technology is to provide a new and improved ambidextrous locking clamp system that has a low cost of manufacturing with regard to both materials and labor, and which accordingly is then susceptible of low prices of sale to the consuming public, thereby making such ambidextrous locking clamp system economically available to the buying public.

Still another embodiment of the present technology is to provide a new ambidextrous locking clamp system that provides in the apparatuses and methods of the prior art some of the advantages thereof, while simultaneously overcoming some of the disadvantages normally associated therewith.

Lastly, it is an object of the present technology to provide a new and improved method of using the ambidextrous locking clamp system by engaging the first and second member ratcheting teeth with each other by an engaging motion provided by the operating hand of a user until the ratcheting teeth overlap one another in succession to a user desired tension. The engaging motion is produced by moving the first and second finger engaging members toward each other. The ratcheting teeth are disengaged by a disengaging motion perpendicular to the engaging motion resulting in moving the first and second latching members away from each other when an opposing force. The disengaging motion is produced by move the first and second members in opposite directions when an opposing force is applied to the first and second finger engaging members by pushing with a thumb of the operating hand of the user on at least one of the first and second finger engaging members and pulling with at least one finger of the operating hand on the other of the first and second finger engaging members thereby slidably separating the ratcheting teeth out of engagement.

These together with other embodiments of the technology, along with the various features of novelty that characterize the technology, are pointed out with particularity in the claims annexed to and forming a part of this disclosure. For a better understanding of the technology, its operating advantages and the specific embodiments attained by its uses, reference should be had to the accompanying drawings and descriptive matter in which there are illustrated preferred embodiments of the technology.

### BRIEF DESCRIPTION OF THE DRAWINGS

The technology will be better understood and embodiments other than those set forth above will become apparent when consideration is given to the following detailed description thereof. Such description makes reference to the annexed drawings wherein:
FIG. 1 is a front plane view of the ambidextrous locking clamp system constructed in accordance with the principles of the present technology.
FIG. 2 is an enlarged front plane view of the ambidextrous locking clamp system in an alternate configuration of the present technology.
FIG. 3 is an exploded front plane view of the ambidextrous locking clamp system of the present technology.
FIG. 4 is a cross-sectional view of the embodiment in FIG. 4 taken along cross-section line 4-4.
FIG. 5 is a cross-sectional view of the embodiment in FIG. 4 taken along cross-section line 5-5.
FIG. 6 is a front plane view of a second alternate embodiment of the ambidextrous locking clamp system of the present technology.
FIG. 7 is an enlarged cross-sectional view of the embodiment in FIG. 6 taken along cross-section line 7-7.
FIG. 8 is an enlarged cross-sectional view of the embodiment in FIG. 6 taken along cross-section line 8-8.
FIG. 9 is an enlarged cross-sectional view o of the embodiment in FIG. 7 taken along cross-section line 9-9.
FIG. 10 is a front plane view of a third alternate embodiment of the ambidextrous locking clamp system of the present technology.
FIG. 11 is an exploded front plane view of the embodiment in FIG. 10.
FIG. 12 is an enlarged cross-sectional view of the embodiment in FIG. 10 taken along cross-section line 12-12.
FIG. 13 is an enlarged cross-sectional view of the embodiment in FIG. 12 taken along cross-section line 13-13.
FIG. 14 is a front plane view of a fourth alternate embodiment of the present technology.
FIG. 15 is an enlarged cross-sectional view of the embodiment in FIG. 14.
FIG. 16 is a front plane view of a fifth alternate embodiment of the present technology.
FIG. 17 is an enlarged cross-sectional view of the embodiment in FIG. 16.
FIG. 18 is a front plane view of a sixth alternate embodiment of the present technology.
FIG. 19 is an enlarged cross-sectional view of the embodiment in 18.
FIG. 20 is a front plane view of a seventh alternate embodiment of the present technology.
FIG. 21 is an enlarged cross-sectional view of the embodiment in FIG. 20.
FIG. 22 is a front plane view of an eighth alternate embodiment of the present technology.
FIG. 23 is an enlarged cross-sectional view of the embodiment in FIG. 22.
FIG. 24 is a front plane view of a ninth alternate embodiment of the present technology.
FIG. 25 is an enlarged cross-sectional view of the embodiment in FIG. 25.
FIG. 26 is a front plane view of a tenth alternate embodiment of the present technology.
FIG. 27 is an enlarged cross-sectional view of the embodiment in FIG. 26.
FIG. 28 is a front plane view of the ambidextrous locking clamp system constructed in accordance with the principles of the present technology.
FIG. 29 is an enlarged front plane view of the ratcheting teeth in an engaging configuration of the present technology.
FIG. 30 is an enlarged bottom elevational view of the ratcheting teeth in a disengaging configuration of the present technology.
FIG. 31 is a front plane view of an eleventh alternate embodiment of the present technology.
FIG. 32 is an enlarged cross-sectional view of the embodiment in FIG. 31.
FIG. 33 is an enlarged bottom elevational view of the ratcheting teeth in a disengaging configuration of the embodiment in FIG. 31.
FIG. 34 is a front plane view of an alternate embodiment of the present technology.
FIG. 35 is a perspective view of the second finger engaging member and latching member of the embodiment in FIG. 34.
FIG. 36 is an enlarged bottom elevational view of the ratcheting teeth in a disengaging configuration of the embodiment in FIG. 34.
FIG. 37 is a front plane view of an alternate embodiment of the present technology.
FIG. 38 is a side plane view of the alternate embodiment in FIG. 37.
FIG. 39 is an enlarged cross-sectional view of the embodiment in FIG. 39 taken along cross-section line 39-39.
FIG. 40 is an enlarged bottom elevational view of the ratcheting teeth in a disengaging configuration of the embodiment in FIG. 37.
FIG. 41 is a front plane view of an alternate embodiment of the present technology.
FIG. 42 is a side plane view of the alternate embodiment in FIG. 41.
FIG. 43 is a perspective view of an alternate embodiment working head section of the present technology.
FIG. 44 is a front plane view of the alternate embodiment in FIG. 43 with the working head in an open configuration.
FIG. 45 is a cross-sectional view of the embodiment in FIG. 44 taken along cross-section line 45-45.
FIG. 46 is a front plane view of the alternate embodiment in FIG. 43 with the working head in a closed configuration.
FIG. 47 is a cross-sectional view of the embodiment in FIG. 46 taken along cross-section line 47-47.
FIG. 48 is a cross-sectional view of the embodiment in FIG. 47 taken along cross-section line 48-48.

The same reference numerals refer to the same parts throughout the various FIGS.

### DETAILED DESCRIPTION OF THE PRESENT TECHNOLOGY

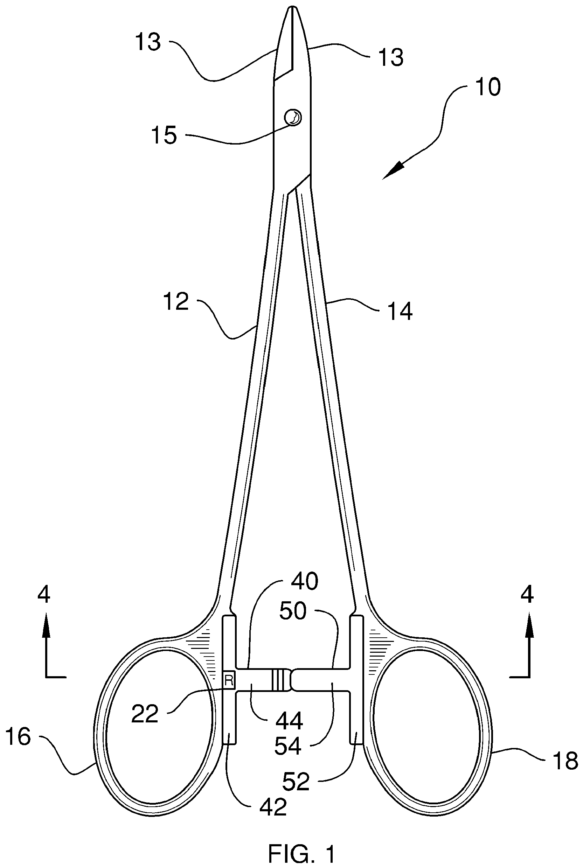

Referring now to the drawings and particularly to FIGS. 1-48, a first embodiment of the ambidextrous locking clamp system of the present technology is shown and generally designated by the reference numeral **10.**

In FIG. 1, a new and improved ambidextrous locking clamp system **10** of the present technology for allowing the use of a hand operated device by a right or left handed user is illustrated and will be described. More particularly, the ambidextrous locking clamp system **10** has a first elongated member **12** and a second elongated member **14** each having a working head **13,** wherein the working heads can be, but not limited to, a gripping jaw or a cutting edge. The second elongated member **14** is connected to the first elongated member **12** via a hinge **15.** The first and second elongated members **12, 14** each has a corresponding finger engaging member **16, 18** located opposite of their respective working heads **13,** wherein each finger engaging member has an indicator **22, 32** for identifying a first and second side of the ambidextrous locking clamp system. Additionally, a first latching member **40** is removably attached to the finger engaging members **16, 18** and a second latching member **50** is removably attached to the finger engaging members **16, 18.** The first and second elongated members **12, 14** can be made from any suitable material having reflex memory.

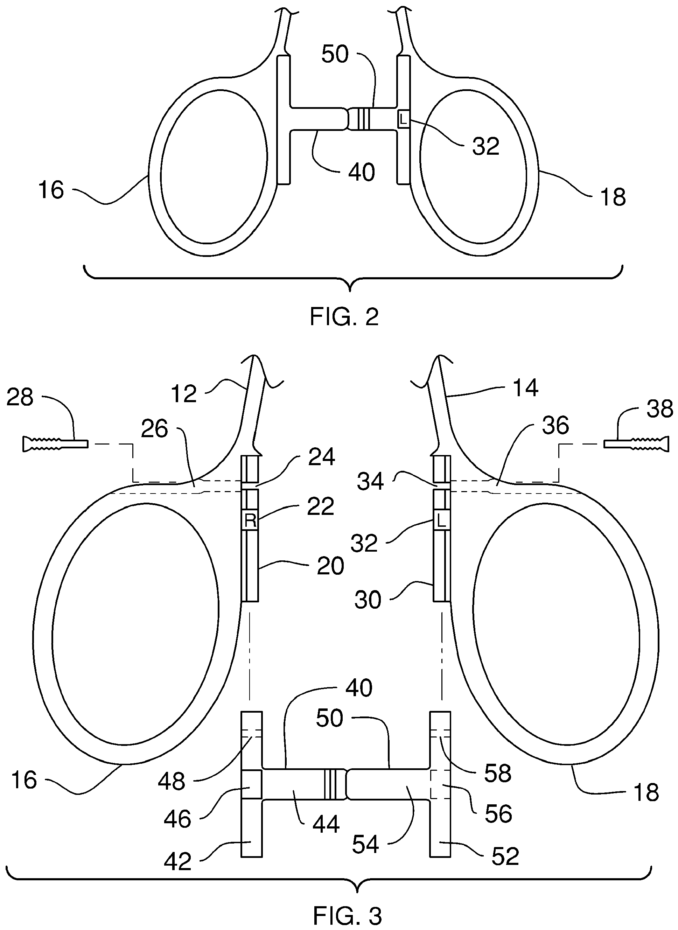

The indicators **22, 32** will have a marking or indicia thereon, such as but limited to an "L" and "R" to indicate the configuration of the ambidextrous locking clamp **10.** Indicator **22** is located on a protrusion **20** extending out from the first finger engaging member **16,** and indicator **32** is located on a protrusion **30** extending out from the second finger engaging member **18.** The indicators **22, 32** are intended to separately and independently identify the first and second elongated members **12, 14** of the ambidextrous locking clamp system **10** respectively attached thereon, so a user can distinguish between the left and right, as best illustrated in FIGS. 1 and 2.

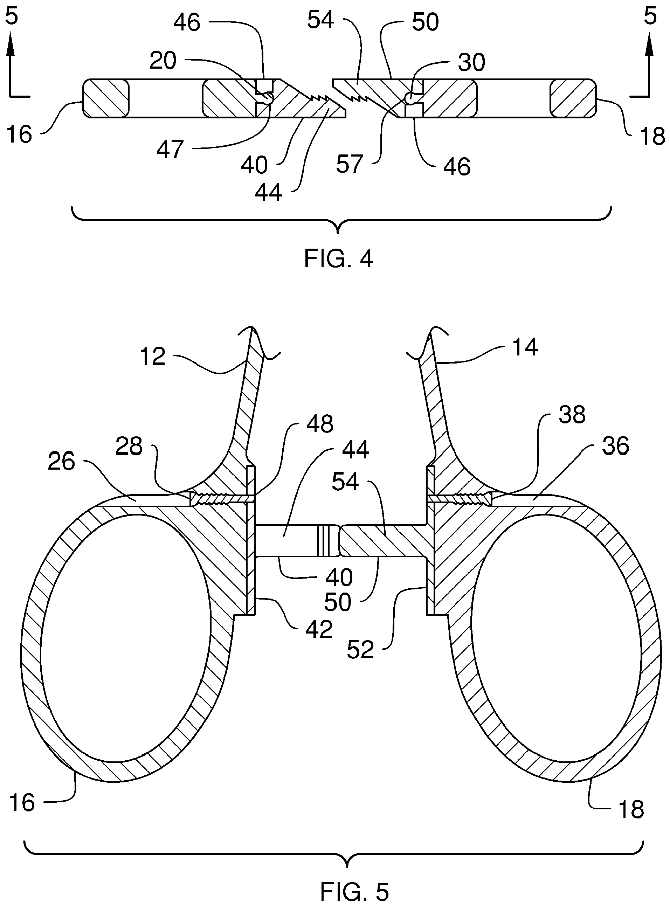

The first and second latching members **40, 50** each have an elongated base **42, 52,** a ratcheting head **44, 54** extending out from their respective elongated base, an opening **46, 56** for viewing the indicator **22, 32** therebelow, and an aperture **48, 58** adapted and configured to receive a threaded retaining pin **28, 38.** The ratcheting heads **44, 54** are substantially perpendicular to their respective elongated bases **42, 52,** thereby forming a generally T-shaped configuration. The retaining pins **28, 38** are securely retained within a bore **26, 36** defined through the finger engaging members **16, 18,** and also extend through the apertures **48, 58** thereby prevent the latching members **40, 50** from being removed from their respective protrusions **20, 30.** The bores **26, 36** can be partially or completely threaded so as to threadably receive the retaining pins **28, 38,** as best illustrated in FIGS. 3 and 5. The retaining pins **28, 38** can each have a non-threaded tip configured to be received through the apertures **48, 58** of the latching members **40, 50.** The ratcheting heads **44, 54** feature a plurality of teeth thereon, which are adapted to join and lock together when engaged by squeezing the finger engaging members **16, 18** together. The teeth are able to disengage when pulled apart by the flexing of the first and second elongated members **12, 14** when an opposing force is applied to the finger engaging members **16, 18.**

The protrusion **20** features a notch **24** aligned with the bore **26.** The bore **26** and the notch **24** are adapted and configured to receive the retaining pin **28, 38** therethrough. The retaining pin **28** is threaded and has a non-threaded tip, wherein the tip is adapted to be received through the notch **24.** The protrusion **20** is adapted to slidably receive latching members **40, 50.** The protrusion **30** features a notch **34** aligned with the bore **36.** The bore **36** and the notch **34** are adapted and configured to receive the retaining pin **28, 38** therethrough. The retaining pin **38** is threaded and has a non-threaded tip, wherein the tip is adapted to be received through the notch **34.** The protrusion **30** is adapted to slidably receive latching members **40, 50.** FIG. 3 is an exploded view best illustrating the above configuration. It can be appreciated that retaining pins **28, 38** are identical and interchangeable

The elongated base **42, 52** of the first and second latching members **40, 50** each have a channel **47, 57** running the length of the elongated base. The channels **47, 57** are adapted and configured to slide on and be retained by the protrusions **20, 30** extending out from the finger engaging members **16, 18.** The configuration of the channels **47, 57** and the protrusions **20, 30** allow the first and second latching members **40, 50** to slide over the protrusion, but at the same time not allowing the latching members to be pulled off the protrusions in a direction perpendicular to the sliding motion. FIGS. 4 and 5 best illustrate one possible example of the channel and protrusion configuration.

The first and second latching members **40, 50** are symmetrical so that they may be removed, inverted and then replaced, thereby changing the orientation of the latching members and allowing a right or left handed user to operate the device **10.** Furthermore, other configurations of the first and second latching members **40, 50** maybe used in place of the above described latching members.

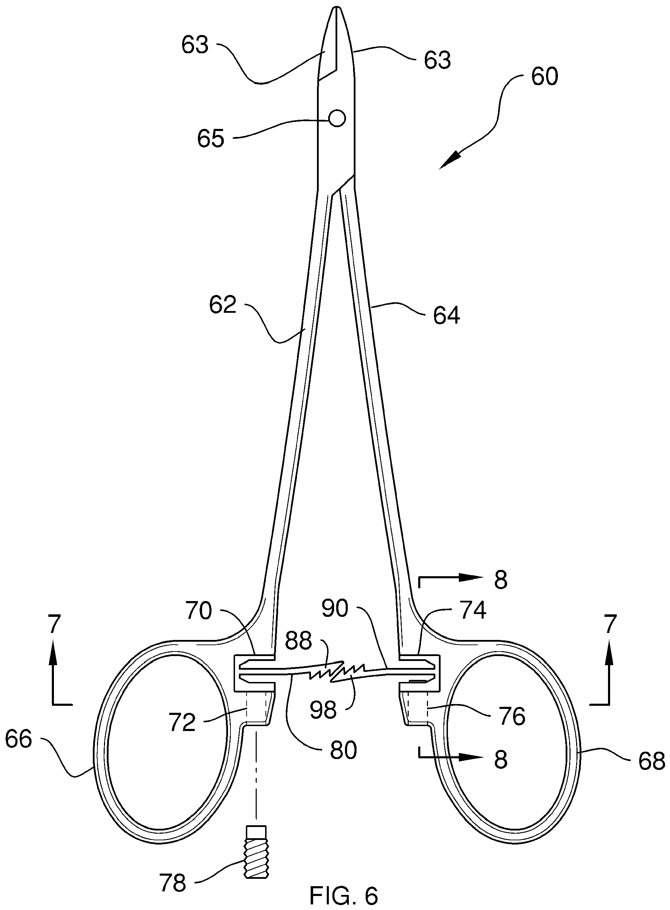

Referring now to FIG. 6, a second alternate embodiment of the ambidextrous locking clamp system of the present technology is shown and generally designated by the reference numeral **60.** More particularly, the ambidextrous locking clamp system **60** has a first elongated member **62** and a second elongated member **64** each having a working head **63,** wherein the working heads can be, but not limited to, a gripping jaw or a cutting edge. The second elongated member **64** is connected to the first elongate member **62** via a hinge **65.** The first and second elongated members **62, 64** each have a corresponding finger engaging member **66, 68** located opposite of their respective working heads **63.** A first latching member **80** and a second latching member **90** are interchangeably and slidably received in a notch **70** located in the first finger engaging member **66,** and in a notch **74** located in the second finger engaging member **68.** The notches **70, 74** are orientated so as to face each other. Additionally, a retaining pin **78** is used to secure the latching members **80, 90** in their respective notches **70, 74,** through a threaded bore **72** located adjacent the notch **70** and a threaded bore **76** located adjacent the notch **74.** The threaded bores **72, 76** are substantially perpendicular with their respective adjacent notches **70, 74.** The threaded bores **72, 76** are in communication with their respective notches **70, 74.**

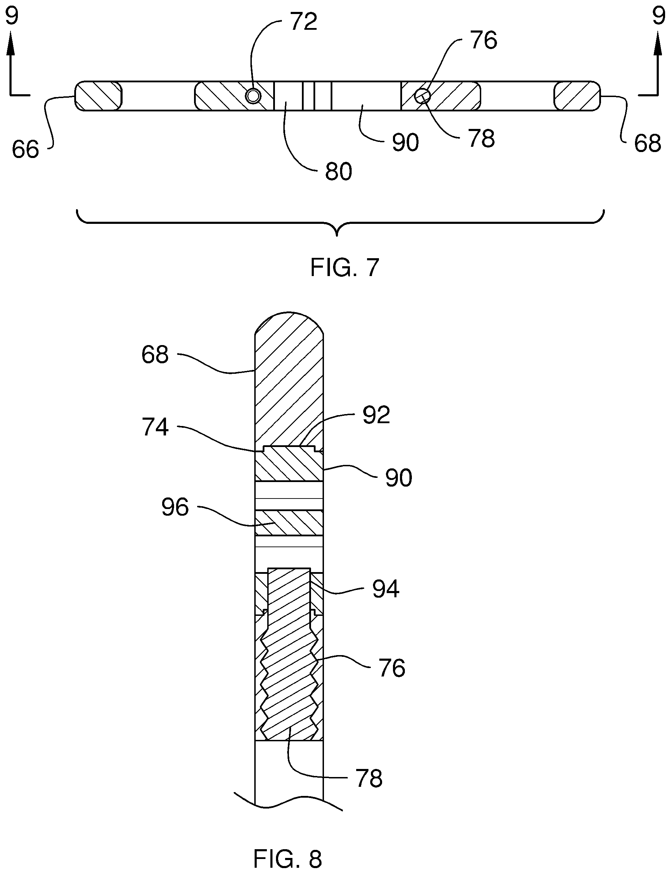

FIG. 7 illustrates the bore **72** without the retaining pin, while bore **76** has the retaining pin 78 therein.

It should be understood that only finger engaging member **68** is shown in FIG. 8 and described herewith, and that latching members **80, 90** can be used with either finger engaging members **66, 68.** The latching member **90** features a detent **92** on a first side of the latching member that corresponds to a top side of the notch **74.** The detent **92** protrudes into the corresponding top side of the notch **74,** allowing the first and second latching members **80, 90** to slide in the notch **74,** but at the same time not allowing the latching members to be pulled out of the notch **74** in a direction perpendicular to the sliding motion. FIG. 8 also illustrates the retaining pin **78** threadably retained in the threaded bore **76.** The retaining pin **78** has a non-threaded tip which is received within an aperture **94** located through a second side of the latching member **90** opposite the detent **92.** The retaining pin **78** secures the latching member **90** in the notch **74,** preventing the latching member from being removed from the slot. The second side of the latch member **90** is configured to receive a detent protruding from a bottom side of the notch **74.**

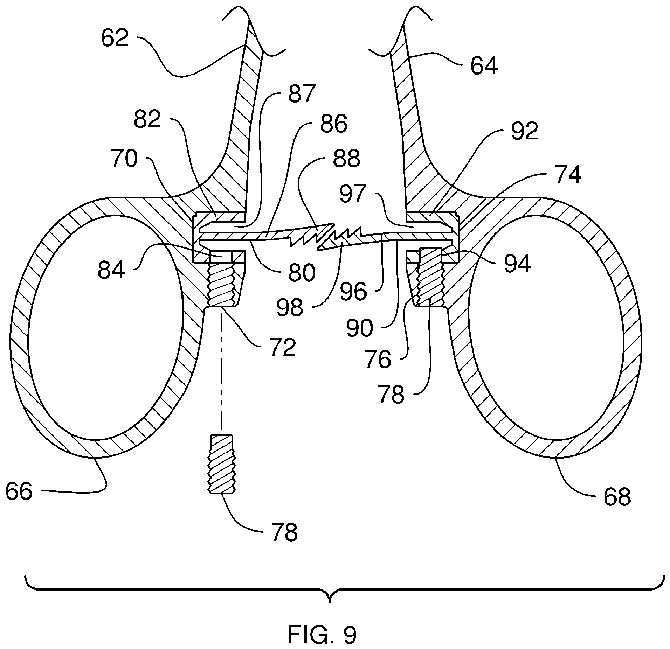

FIG. 9 best illustrates one possible configuration of the first and second latching members **80, 90** in relation to their respective finger engaging members **66, 68.** The first latching member **80** has an elongated flexible arm **86** with a ratcheting head **88** featuring a plurality of ratcheting teeth thereon, a detent **82,** and an aperture **84.** The detent **82** protrudes into a corresponding top side of notch **70, 74,** as described above. The aperture **84** is located on a second side opposite the detent **82** and is aligned with the threaded bore **72, 76** when positioned in its respective notch **70, 74.** The aperture **84** is adapted to receive the tip of the retaining pin **78** therethrough or therein when the retaining pin is threaded in the bore **72, 76.** The flexible arm **86** is positioned between the detent **82** and the aperture **84** sides of the latching member **80** so as to extend through a first latching member notch **87** defined in the first latching member **80** between the detent **82** and the aperture **84** sides.

The second latching member **90** has an elongated flexible arm **96** with a ratcheting head **98** featuring a plurality of teeth thereon, the detent **92,** and the aperture **94.** The detent **92** protrudes into a corresponding top side of notch **70, 74,** as described above. The aperture **94** is located opposite the detent **92** and is aligned with the threaded bore **72, 76** when positioned in its respective notch **70, 74.** The aperture **94** is adapted to receive the tip of the retaining pin **78** therethrough or therein when the retaining pin is threaded in the bore **72, 76.** The flexible arm **96** is positioned between the detent **92** and the aperture **94** sides of the latching member **90** so as to extend through a second latching member notch **97** defined in the second latching member **90** between the detent **92** and the aperture **94** sides. The ratcheting heads **88, 98** are adapted to join and lock together when engaged by squeezing the finger engaging members **66, 68** together. The teeth are able to disengage when pulled apart by flexing the first and second elongated members **62, 64** when an opposing perpendicular force in either direction is applied to the finger engaging members **66, 68** by pushing with the thumb and pulling with the fingers of the operating hand thereby separating the parallel engaged teeth.

It can be appreciated that retaining pins **78** are identical and interchangeable, and that the first and second latching members **80, 90** are interchangeable with notches **70, 74.** It can also be appreciated that the ambidextrous locking clamp system **60** can be used with either the left or right hand with identical methods of separating and disengaging the teeth of ratcheting heads **88, 98.**

The first and second latching members **80, 90** are symmetrical so that they may be removed and interchanged with each other, and then replaced, thereby changing the orientation of the latching members of device **60.**

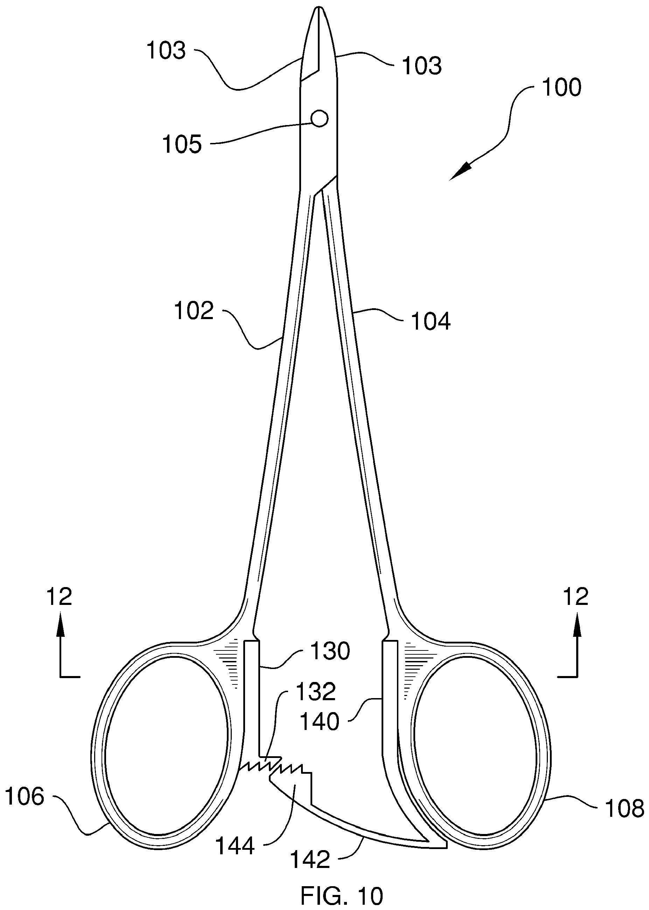

Referring now to FIG. 10, a third alternate embodiment of the ambidextrous locking clamp system of the present technology is shown and generally designated by the reference numeral **100.** More particularly, the ambidextrous locking clamp system **100** has a first elongated member **102** and a second elongated member **104** each having a working head **103,** wherein the working heads can be, but not limited to, a gripping jaw or a cutting edge. The second elongated member **104** is connected to the first elongate member **102** via a hinge **105.** The first and second elongated members **102, 104** have a corresponding finger engaging member **106, 108** located opposite of the working heads **103,** wherein each finger engaging member can slidably receive a first latching member **130** or a second latching member **140.** The first latching member **130** has a ratcheting head **132** featuring ratcheting teeth thereon, and the second latching member **140** has a flexible arm **142** extending out therefrom, and a ratcheting head **144** located at the free end of the flexible arm **142.** The ratcheting head **144** features ratcheting teeth thereon adapted to engage with the ratcheting teeth of the ratcheting head **132.**

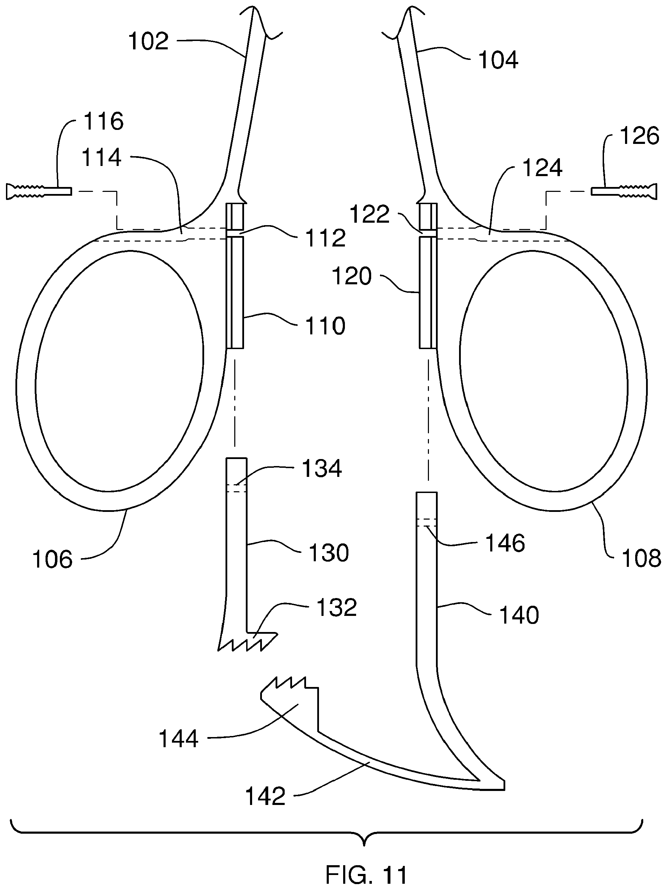

The finger engaging member **106** has a threaded bore **114,** and a protrusion **110** extending out from the finger engaging member **106** perpendicular to the threaded bore **114.** The protrusion **110** features a notch **112** aligned with the bore **114.** The bore **114** and the notch **112** are adapted and configured to receive a retaining pin **116** therethrough. The retaining pin **116** is threaded and has a non-threaded tip, wherein the tip is adapted to be received through the notch **112.** The protrusion **110** is adapted to slidably receive latching members **130, 140.** The finger engaging member **108** has a threaded bore **124,** and a protrusion **120** extending out from the finger engaging member **108** perpendicular to the threaded bore **124.** The protrusion **120** features a notch **122** aligned with the bore **124.** The bore **124** and the notch **122** are adapted and configured to receive a retaining pin **126** therethrough. The retaining pin **126** is threaded and has a non-threaded tip, wherein the tip is adapted to be received through the notch **122.** The protrusion **120** is adapted to slidably receive latching members **130, 140.** It can be appreciated that retaining pins **116, 126** are identical and interchangeable. FIG. 11 is an exploded view best illustrating the above configuration.

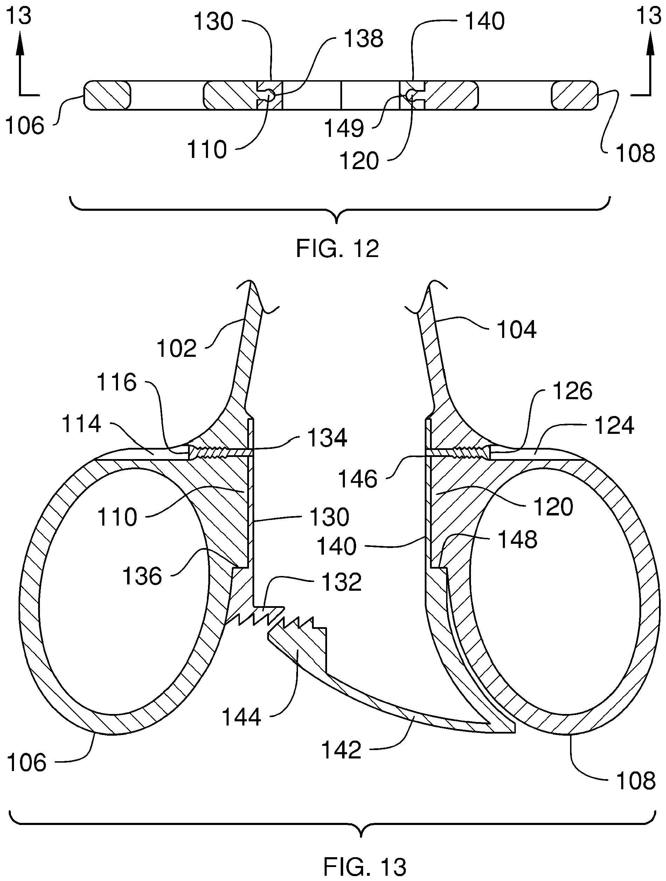

The latching members **130, 140** each have a channel **138, 149** running the length of their respective latching members. The channels **138, 149** are adapted and configured to slide on and be retained by the protrusions **110, 120** extending out from their respective finger engaging members **106,108.** The configuration of the channels **138, 149** and the protrusions **110, 120** allow the latching members **130, 140** to slide over the protrusion, but at the same time not allowing the latching members to be pulled off the protrusions in a direction perpendicular to the sliding motion. FIG. 12 best illustrates one possible example of the channel and protrusion configuration.

FIG. 13 best illustrates one possible configuration of the first and second latching members **130, 140** in relation to their respective finger engaging members **106, 108.** The first latching member **130** has the ratcheting head **132** featuring a plurality of ratcheting teeth thereon, a stop **136,** and an aperture **134.** The stop **136** is perpendicular to the longitudinal axis of the latching member **130** and it is adapted to abut against a free end of it respective protrusion **110, 120.** The aperture **134** is located opposite the stop **136** and is aligned with the threaded bore **114, 124** and the notch **112,122** when positioned on its respective protrusion **110, 120.** The aperture **134** is adapted to receive the tip of the retaining pin **116, 126** therethrough or therein when the retaining pin is threaded in the bore **114, 124,** through notch **112, 122,** and through aperture **134.** The second latching member **140** has the flexible arm **142,** the ratcheting head **144** featuring a plurality of ratcheting teeth thereon, a stop **148,** and an aperture **146.** The stop **148** is perpendicular to the longitudinal axis of the latching member **140** and it is adapted to abut against a free end of it respective protrusion **110, 120.** The aperture **146** is located opposite the stop **148** and is aligned with the threaded bore **114, 124** and the notch **112,122** when positioned on its respective protrusion **110,120.** The aperture **146** is adapted to receive the tip of the retaining pin **116, 126** therethrough or therein when the retaining pin is threaded in the bore **114, 124,** and through notch **112, 122** and aperture **146.** The flexible arm **142** extends out from a section of the latching member **140** extending past the ratcheting head **132** of the first latching member **130,** when both latching members **130, 140** are attached to their respective protrusions **110, 120.** The flexible arm **142** has a generally arcuate shape curving upwardly toward the ratcheting head **132** of the first latching member **130.** This extension of the second latching member **140** has a shape that corresponds to the shape of the finger engaging member **106,108.** The ratcheting head **144** is attached to the free end of the flexible arm **142,** thereby allowing the ratcheting head **144** to free travel with the flexing of the flexible arm **142.** The ratcheting heads **132, 144** are adapted to join and lock together when engaged by squeezing the finger engaging members **106, 108** together. The teeth are able to disengage when pulled apart by flexing the first and second elongated members **102, 104** when an opposing perpendicular force is applied to the finger engaging members **106, 108** by pushing with the thumb and pulling with the fingers of the operating hand thereby separating the parallel engaged teeth.

It can be appreciated that retaining pins **116, 126** are identical and interchangeable, and that the channels **138, 149** of first and second latching members **130, 140** are identical interchangeable with protrusions **110, 120.** It can also be appreciated that the ambidextrous locking clamp system **100** can be used with either the left or right hand with identical methods of separating and disengaging the teeth of ratcheting heads **132, 144.**

The channels **138, 149,** apertures **134, 146,** and stop **136, 148** of the first and second latching members **130, 140** are symmetrical so that they may be removed, inverted and then replaced, thereby changing the orientation of the latching members of device **100.** Furthermore, other configurations of the first and second latching members **130, 140** may be used in place of the above described latching members.

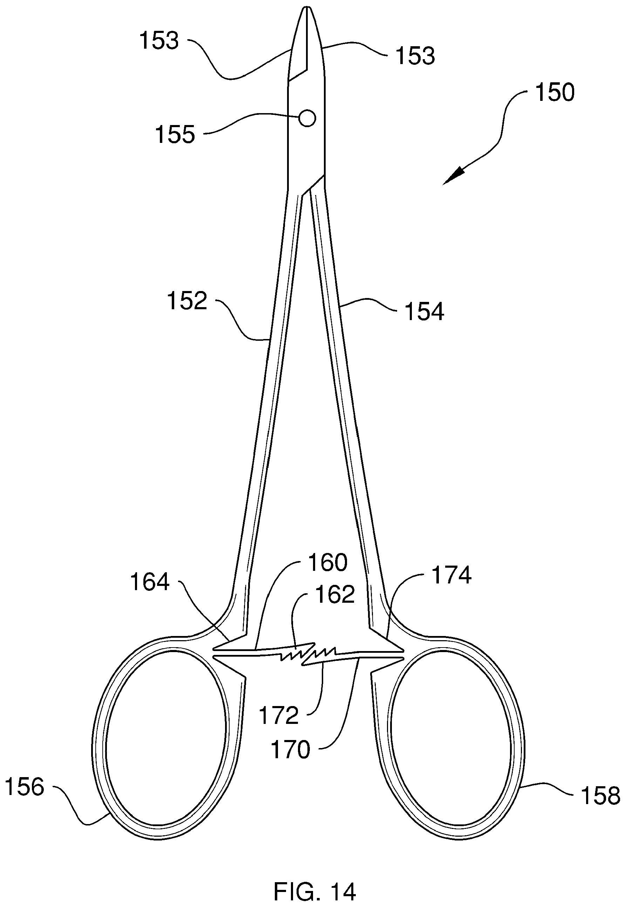

Referring now to FIG. 14, a fourth alternate embodiment of the ambidextrous locking clamp system of the present technology is shown and generally designated by the reference numeral **150.** More particularly, the ambidextrous locking clamp system **150** has a first elongated member **152** and a second elongated member **154** each having a working head **153,** wherein the working heads can be, but not limited to, a gripping jaw or a cutting edge. The second elongated member **154** is connected to the first elongate member **152** via a hinge **155.** The first and second elongated members **152, 154** each have a corresponding finger engaging member **156, 158** located opposite of their respective working heads **153.** The first finger engaging member **156** has a latching member **160** extending out from a notch **164,** and a ratcheting head **162** located at the free end of the latching member **160.** The ratcheting head **162** features ratcheting teeth thereon. The second finger engaging member **158** has a latching member **170** extending out from a notch **174,** and a ratcheting head **172** located at the free end of the latching member **170.** The ratcheting head **172** features ratcheting teeth thereon adapted to engage with the ratcheting teeth of the ratcheting head **162** when the first and second finger engaging members **156, 158** are squeezed together.

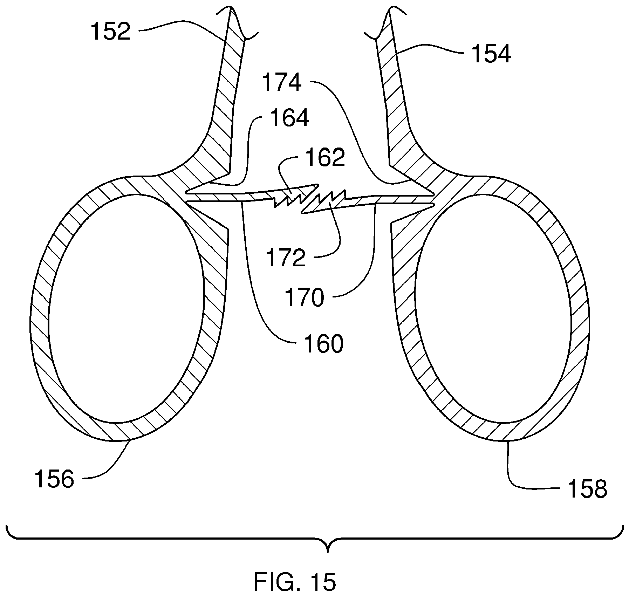

As illustrated in FIG. 15, the first and second finger engaging members **156, 158,** the latching members **160, 170,** and the notches **164, 174** are symmetrical and mirror images of each other. The latching member **160** of the first finger engaging member **156** is a flexible arm that extends out from the notch **164** toward the second finger engaging member **158.** The notch **164** can have any geometric shape, but preferably a V-shape with the latching member **160** extending out from the central interior of the V-shaped notch. The latching member **170** of the second finger engaging member **158** is a flexible arm that extends out from the notch **174** toward the first finger engaging member **156.** The notch **174** can have any geometric shape, but preferably a V-shape with the latching member **170** extending out from the central interior of the V-shaped notch.

The ratcheting heads **162, 172** are adapted to join and lock together when engaged by squeezing the finger engaging members **156, 158** together. The teeth are able to disengage when pulled apart by flexing the first and second elongated members **152, 154** when an opposing force is applied to the finger engaging members **156, 158** in a perpendicular movement in either direction by pushing with the thumb and pulling with the fingers of the operating hand thereby separating the parallel engaged teeth.

It can be appreciated that the ambidextrous locking clamp system **150** can be used with either the left or right hand with identical methods of separating and disengaging the teeth of ratcheting heads **162, 172** and/or with the flexing of the elongated members in a perpendicular movement in either direction.

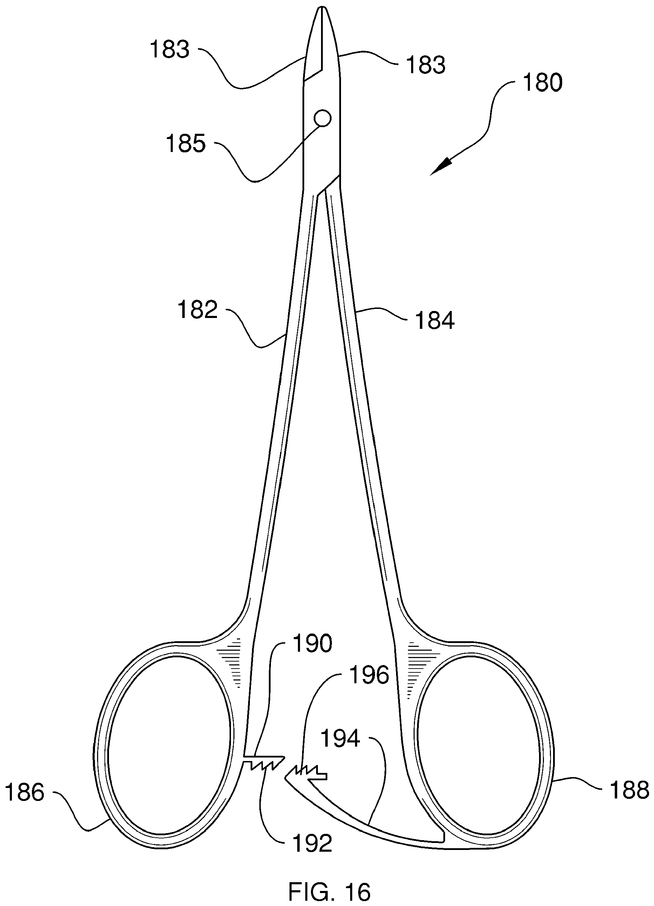

Referring now to FIG. 16, a fifth alternate embodiment of the ambidextrous locking clamp system of the present technology is shown and generally designated by the reference numeral **180.** More particularly, the ambidextrous locking clamp system **180** has a first elongated member **182** and a second elongated member **184** each having a working head **183,** wherein the working heads can be, but not limited to, a gripping jaw or a cutting edge. The second elongated member **184** is connected to the first elongate member **182** via a hinge **185.** The first and second elongated members **182, 184** each have a corresponding finger engaging member **186, 188** located opposite of their respective working heads **183.** The first finger engaging member **186** has a latching member **190** extending out therefrom, and a ratcheting head **192** located at the free end of the latching member **190.** The ratcheting head **192** features ratcheting teeth thereon. The second finger engaging member **188** has a latching member **194** extending out therefrom, and a ratcheting head **196** located at the free end of the latching member **194.** The ratcheting head **196** features ratcheting teeth thereon adapted to engage with the ratcheting teeth of the ratcheting head **192** when the first and second finger engaging members **186, 188** are squeezed together.

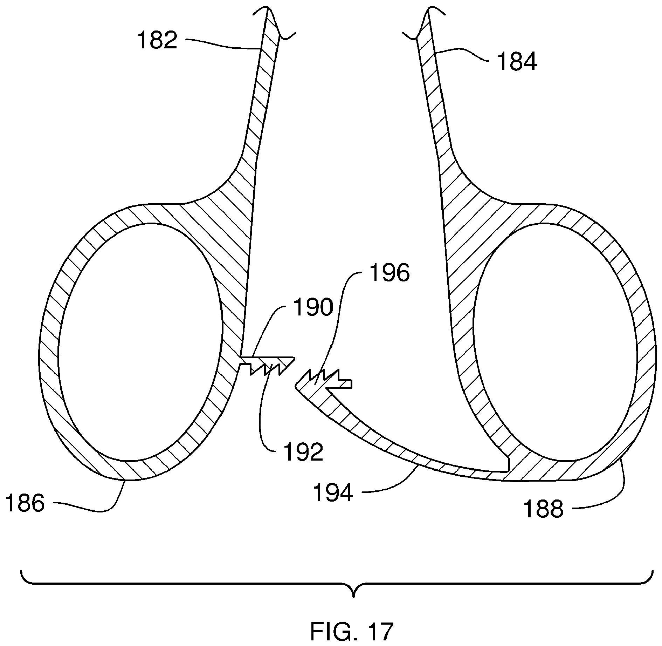

As illustrated in FIG. 17, the latching member **190** of the first finger engaging member **186** extends out from the interior of the first finger engaging member toward the second finger engaging member **188.** The latching member **194** of the second finger engaging member **188** is a flexible arm that extends outwardly and upwardly from the bottom of the second finger engaging member **188** and below the latching member **190** toward the first finger engaging member **186.** The ratcheting head **196** is located on the free end of the flexible arm latching member **194.** The flexible arm latching member **194** tapers with the thickest part being attached to the ratcheting head and the thinnest part being attached to the bottom of the second finger engaging member **188,** and has a generally arcuate shape. Thereby allowing the latching member **194** to have a more degree of flexibility at its second finger engaging member attachment point, and increasing the travel length of ratcheting head **196.**

The ratcheting heads **192, 196** are adapted to join and lock together when engaged by squeezing the finger engaging members **186, 188** together. The teeth are able to disengage when pulled apart by flexing the first and second elongated members **182, 184** when an opposing force is applied to the finger engaging members **186, 188** by pushing with the thumb and pulling with the fingers of the operating hand thereby separating the parallel engaged teeth.

It can be appreciated that the ambidextrous locking clamp system **180** can be used with either the left or right hand with identical methods of separating and disengaging the teeth of ratcheting heads **192, 196.**

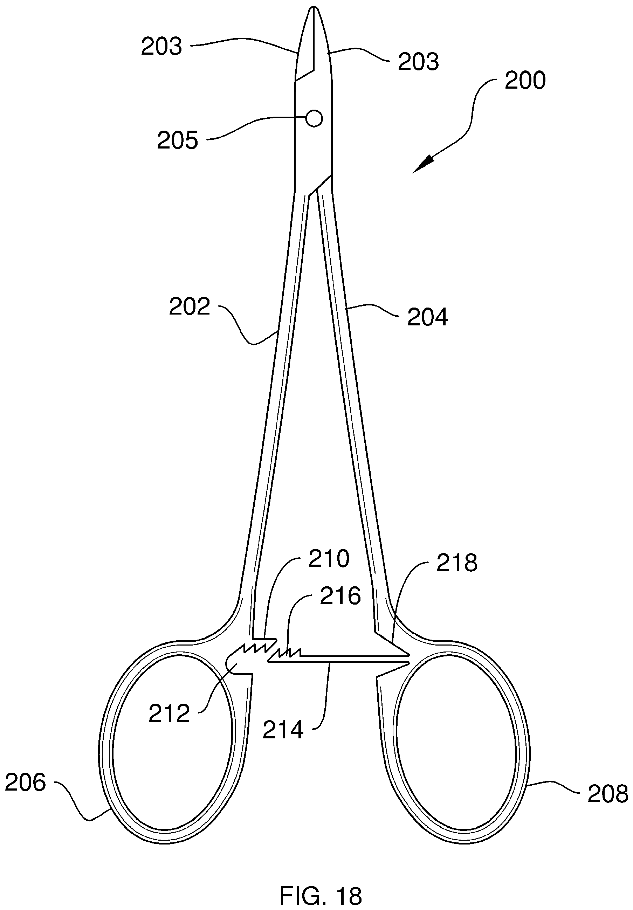

Referring now to FIG. 18, a sixth alternate embodiment of the ambidextrous locking clamp system of the present technology is shown and generally designated by the reference numeral **200.** More particularly, the ambidextrous locking clamp system **200** has a first elongated member **202** and a second elongated member **204** each having a working head **203,** wherein the working heads can be, but not limited to, a gripping jaw or a cutting edge. The second elongated member **204** is connected to the first elongate member **202** via a hinge **205.** The first and second elongated members **202, 204** each have a corresponding finger engaging member **206, 208** located opposite of their respective working heads **203.** The first finger engaging member **206** has a latching member **210** extending out therefrom, and a notch **212** adjacent the latching member **210.** The latching member **210** features ratcheting teeth thereon. The second finger engaging member **208** has a latching member **214** extending out from a notch **218,** and a ratcheting head **216** located at the free end of the latching member **214.** The ratcheting head **216** features ratcheting teeth thereon adapted to engage with the ratcheting teeth of the latching member **210** when the first and second finger engaging members **206, 208** are squeezed together.

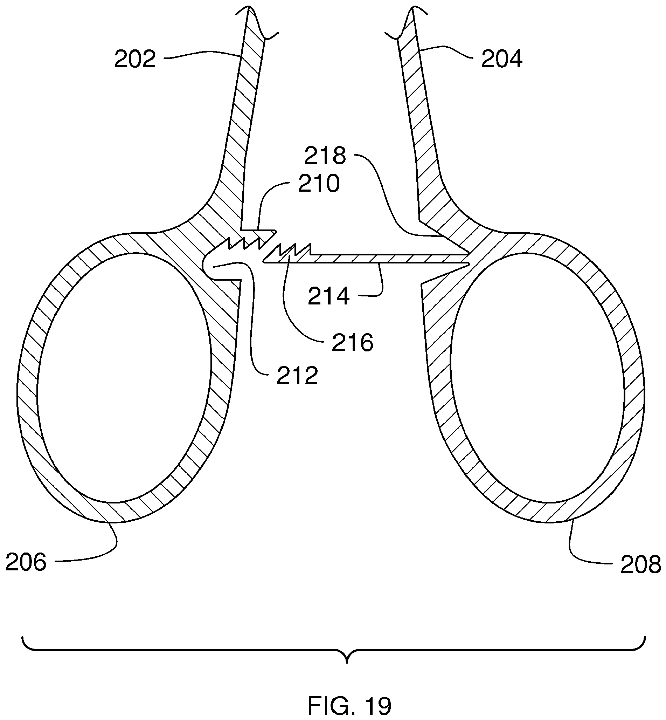

As illustrated in FIG. 19, the latching member **210** of the first finger engaging member **206** extends out from the first finger engaging member toward the second finger engaging member **208.** The notch **212** can have any geometric shape, but preferably a U-shape with the latching member **210** being positioned directly above or below and adjacent to the U-shaped notch. The notch **212** being configured to receive the ratcheting head **216** of the latching member **214** of the second finger engaging member **208,** and allowing for the ratcheting head **216** to disengage from the first latching member **210.** The latching member **214** of the second finger engaging member **208** is a flexible arm that extends out from the notch **218** toward the first finger engaging member **206.** The notch **218** can have any geometric shape, but preferably a V-shape with the latching member **214** extending out from the central interior of the V-shaped notch.

The ratcheting head **216** and the ratcheting teeth of the latching member **210** are adapted to join and lock together when engaged by squeezing the finger engaging members **206, 208** together. The teeth are able to disengage when pulled apart by flexing the first and second elongated members **202, 204** when an opposing force is applied to the finger engaging members **206, 208** by pushing with the thumb and pulling with the fingers of the operating hand thereby separating the parallel engaged teeth.

It can be appreciated that the ambidextrous locking clamp system **200** can be used with either the left or right hand with identical methods of separating and disengaging the teeth of the first latching member **210** and ratcheting head **216.**

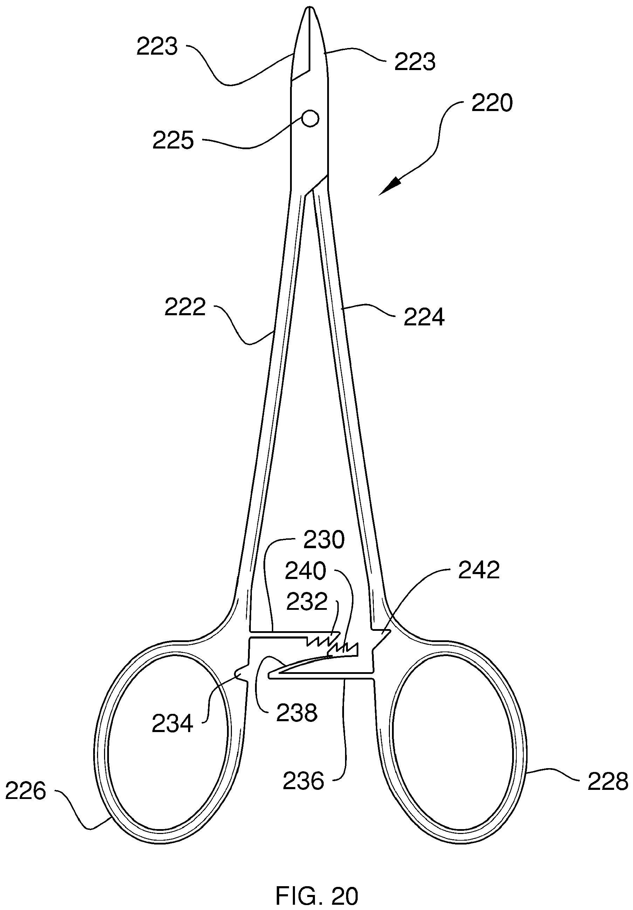

Referring now to FIG. 20, a seventh alternate embodiment of the ambidextrous locking clamp system of the present technology is shown and generally designated by the reference numeral **220.** More particularly, the ambidextrous locking clamp system **220** has a first elongated member **222** and a second elongated member **224** each having a working head **223,** wherein the working heads can be, but not limited to, a gripping jaw or a cutting edge. The second elongated member **224** is connected to the first elongate member **222** via a hinge **225.** The first and second elongated members **222, 224** each have a corresponding finger engaging member **226, 228** located opposite of their respective working heads **223.** The first finger engaging member **226** has a latching member **230** extending out therefrom, a ratcheting head **232** located at the free end of the latching member **230,** and a notch **234.** The ratcheting head **232** features ratcheting teeth thereon. The second finger engaging member **228** has a latching member **236** extending out therefrom, a flexible arm **238,** a ratcheting head **240,** and a notch **242.** The ratcheting head **240** features ratcheting teeth thereon adapted to engage with the ratcheting teeth of the ratcheting head **232** when the first and second finger engaging members **226, 228** are squeezed together.

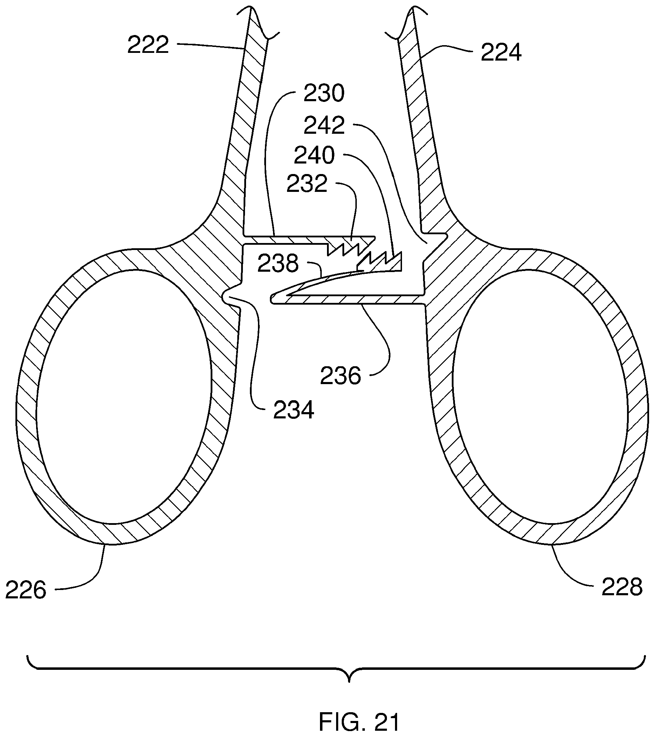

As illustrated in FIG. 21, the latching member **230** of the first finger engaging member **226** extends out from the first finger engaging member toward the second finger engaging member **228.** The notch **234** can have any geometric shape, but preferably a U-shape aligned with the latching member **236.** The latching member **230** is positioned above or below the notch **234.** The notch **234** is configured to receive the flexible arm **238** and the latching member **236** attachment point. The flexible arm **238** is attached to the free end of the latching member **236,** and the ratcheting head **240** is attached to the free end of the flexible arm **238.** The latching member **236** of the second finger engaging member **228** extends out from the second finger engaging member **228** toward the notch **234** of the first finger engaging member **226.** The flexible arm **238** extends inwardly and upwardly from the free end of the latching member **236,** and has an arcuate shape. The flexible arm **238** allows for the free travel of the ratcheting head **240.** The notch **242** is positioned above or below the latching member **236** and is aligned with the latching member **230,** and is adapted and configured to receive the ratcheting head **232** of the latching member **230.** The notch **242** can have any geometric shape, but preferably a shape that corresponds to the shape of the ratcheting head **232** of the latching member **230.**

The ratcheting teeth of the ratcheting head **232** and the ratcheting teeth of the ratcheting head **240** are adapted to join and lock together when engaged by squeezing the finger engaging members **226, 228** together. The teeth are able to disengage when pulled apart by flexing the first and second elongated members **222, 224** when an opposing force is applied to the finger engaging members **226, 228** by pushing with the thumb and pulling with the fingers of the operating hand thereby separating the parallel engaged teeth.

It can be appreciated that the ambidextrous locking clamp system **220** can be used with either the left or right hand with identical methods of separating and disengaging the teeth of ratcheting heads **232, 240.**

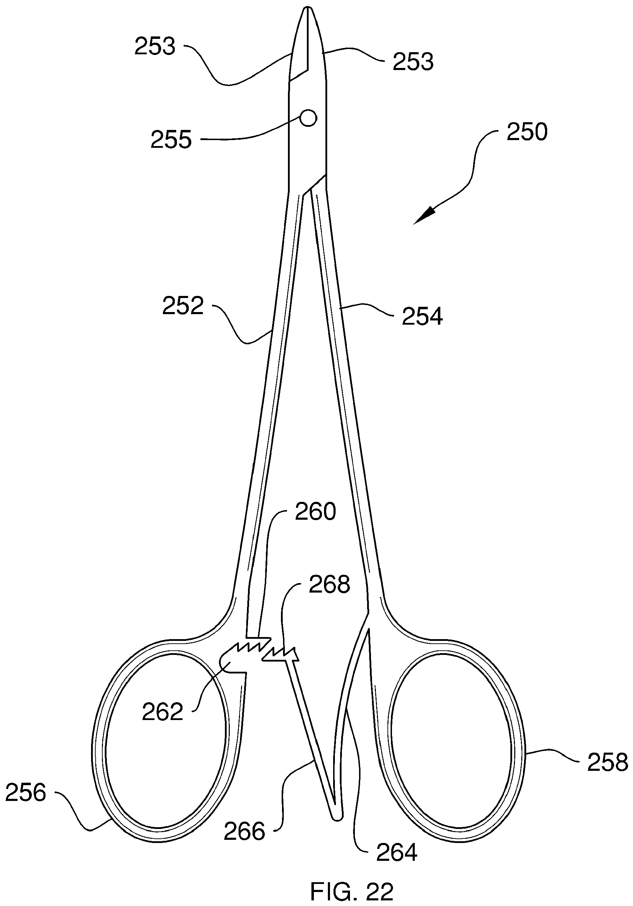

Referring now to FIG. 22, an eighth alternate embodiment of the ambidextrous locking clamp system of the present technology is shown and generally designated by the reference numeral **250.** More particularly, the ambidextrous locking clamp system **250** has a first elongated member **252** and a second elongated member **254** each having a working head **253,** wherein the working heads can be, but not limited to, a gripping jaw or a cutting edge. The second elongated member **254** is connected to the first elongate member **252** via a hinge **255.** The first and second elongated members **252, 254** each have a corresponding finger engaging member **256, 258** located opposite of their respective working heads **253.** The first finger engaging member **256** has a latching member **260** extending out therefrom, and a notch **262** adjacent the latching member **260.** The latching member **260** features ratcheting teeth thereon. The second finger engaging member **258** has a latching member **264** extending out therefrom, an arm **266,** and a ratcheting head **268.** The ratcheting head **268** features ratcheting teeth thereon adapted to engage with the ratcheting teeth of the latching member **260** when the first and second finger engaging members **256, 258** are squeezed together.

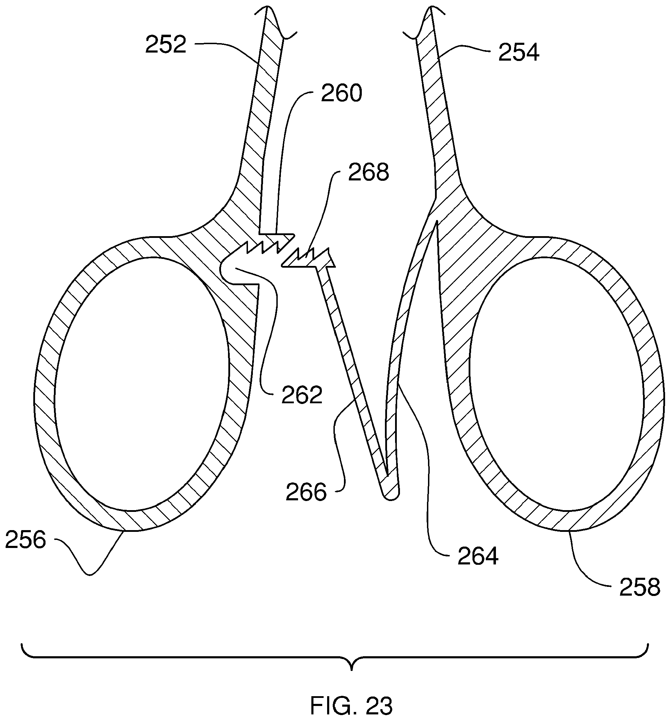

As illustrated in FIG. 23, the latching member **260** of the first finger engaging member **256** extends out from the first finger engaging member toward the second finger engaging member **258.** The notch **262** can have any geometric shape, but preferably a U-shape with the latching member **260** being positioned directly above or below and adjacent to the U-shaped notch. The notch **262** is aligned with the ratcheting head **268** of the latching member **264,** allowing the ratcheting head **268** to disengage from the ratcheting head **260,** while received therein. The latching member **264** of the second finger engaging member **258** extends out from the second finger engaging member toward the first finger engaging member **256.** The arm **266** is attached to the free end of the latching member **264,** and the ratcheting head **268** is attached to the free end of the arm **266.** The latching member **264** and the arm **266** are flexible allowing for the free travel of the ratcheting head **268,** with respect to the second finger engaging member **258.** The latching member **264** extends outwardly and downwardly from the interior of the second finger engaging member **258,** and has a generally arcuate shape. The arm **266** extends outwardly and upwardly from the free end of the latching member **264.**

The ratcheting teeth of the latching member **260** and the ratcheting teeth of the ratcheting head **268** are adapted to join and lock together when engaged by squeezing the finger engaging members **256, 258** together. The teeth are able to disengage when pulled apart by flexing the first and second elongated members **252, 254** when an opposing force is applied to the finger engaging members **256, 258** by pushing with the thumb and pulling with the fingers of the operating hand thereby separating the parallel engaged teeth.

It can be appreciated that the ambidextrous locking clamp system **250** can be used with either the left or right hand with identical methods of separating and disengaging the teeth of the first latching member **260** and the ratcheting head **268.**

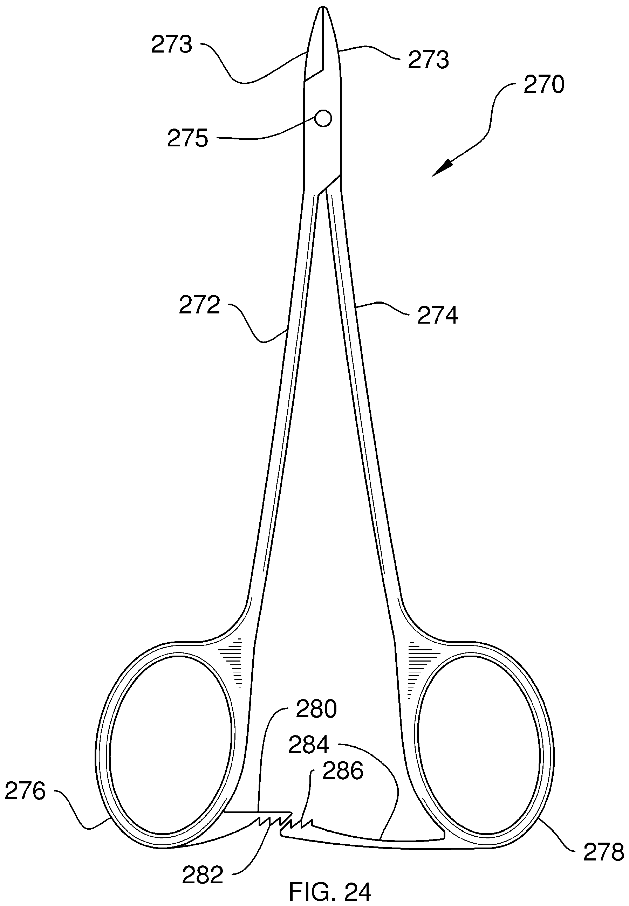

Referring now to FIG. 24, a ninth alternate embodiment of the ambidextrous locking clamp system of the present technology is shown and generally designated by the reference numeral **270.** More particularly, the ambidextrous locking clamp system **270** has a first elongated member **272** and a second elongated member **274** each having a working head **273,** wherein the working heads can be, but not limited to, a gripping jaw or a cutting edge. The second elongated member **274** is connected to the first elongate member **272** via a hinge **275.** The first and second elongated members **272, 274** each have a corresponding finger engaging member **276, 278** located opposite of their respective working heads **273.** The first finger engaging member **272** has a latching member 280 extending out therefrom, and a ratcheting head **282.** The ratcheting head **282** features ratcheting teeth thereon. The second finger engaging member **278** has a latching member **284** extending out therefrom, and a ratcheting head **286.** The ratcheting head **286** features ratcheting teeth thereon adapted to engage with the ratcheting teeth of the ratcheting head **282** when the first and second finger engaging members **276, 278** are squeezed together.

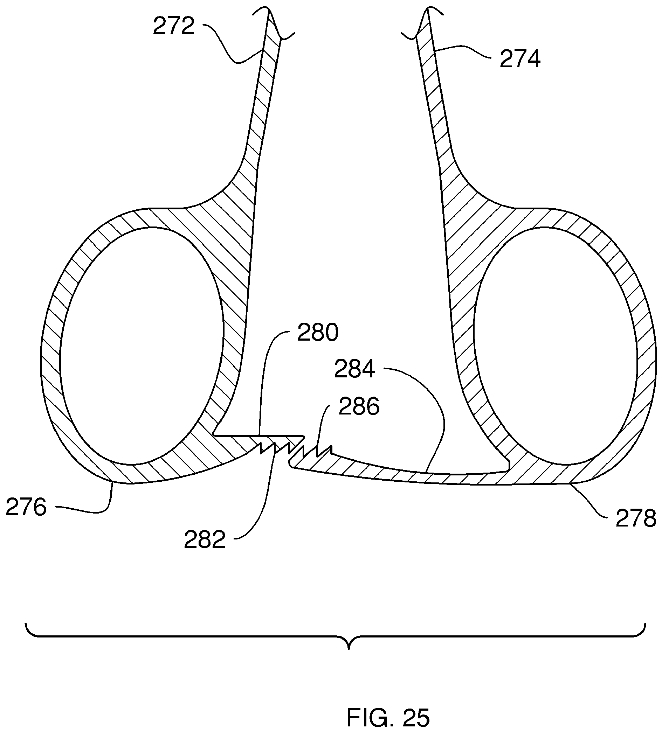

As illustrated in FIG. 25, the latching member **280** of the first finger engaging member **276** extends out from the bottom of the first finger engaging member toward the second finger engaging member **278.** The latching member **284** of the second finger engaging member **278** extends out from the bottom of the second finger engaging member toward the first finger engaging member **276.** The bottom side of the latching member **280** has a generally arcuate shape featuring an upwardly curve so as not to interfere with the movement of ratcheting head **286** of the latching member **284** when engaging or disengaging from ratcheting head **282.** The latching member **280** is thicker at its attachment point to the first finger engaging member **276** than at its attachment point to the ratcheting head **282.** The latching member **284** is a flexible arm, and the ratcheting head **286** is attached to the free end of the flexible arm latching member **284.** The flexible arm latching member **284** allows for the free travel of the ratcheting head **286,** with respect to the second finger engaging member **278.**

The ratcheting teeth of the ratcheting head **282** of latching member **280** and the ratcheting teeth of the ratcheting head **286** are adapted to join and lock together when engaged by squeezing the finger engaging members **276, 278** together. The teeth are able to disengage when pulled apart by flexing the first and second elongated members **272, 274** when an opposing force is applied to the finger engaging members **276, 278** by pushing with the thumb and pulling with the fingers of the operating hand thereby separating the parallel engaged teeth.

It can be appreciated that the ambidextrous locking clamp system **270** can be used with either the left or right hand with identical methods of separating and disengaging the teeth of ratcheting heads **282, 286.**

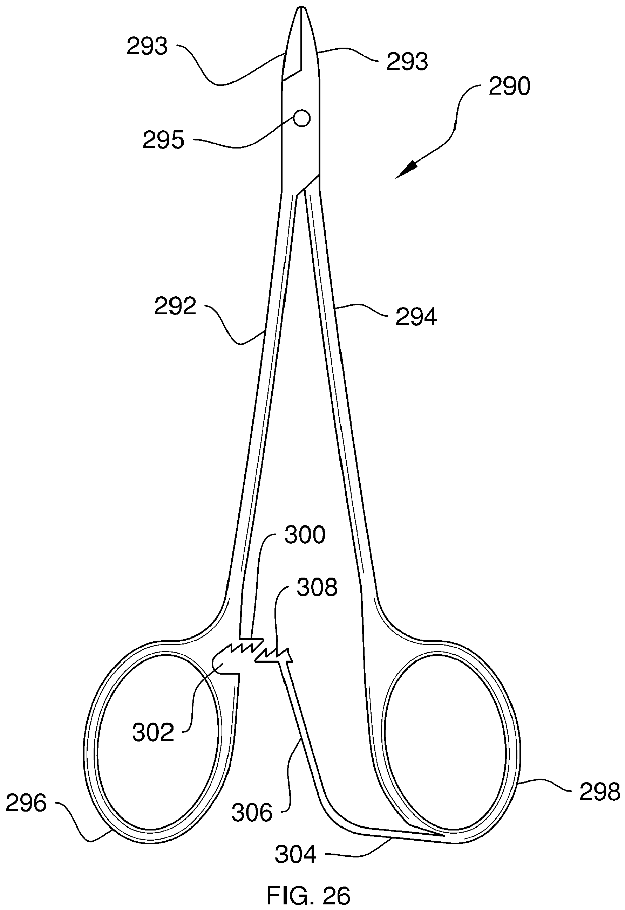

Referring now to FIG. 26, a tenth alternate embodiment of the ambidextrous locking clamp system of the present technology is shown and generally designated by the reference numeral **290.** More particularly, the ambidextrous locking clamp system **290** has a first elongated member **292** and a second elongated member **294** each having a working head **293,** wherein the working heads can be, but not limited to, a gripping jaw or a cutting edge. The second elongated member **294** is connected to the first elongate member **292** via a hinge **295.** The first and second elongated members **292, 294** each have a corresponding finger engaging member **296, 298** located opposite of their respective working heads **293.** The first finger engaging member **296** has a latching member **300** extending out therefrom, and a notch **302** adjacent the latching member **300.** The latching member **300** features ratcheting teeth thereon. The second finger engaging member **298** has a latching member **304** extending out therefrom, an arm **306,** and a ratcheting head **308.** The ratcheting head **308** features ratcheting teeth thereon adapted to engage with the ratcheting teeth of the latching member **300** when the first and second finger engaging members **296, 298** are squeezed together.

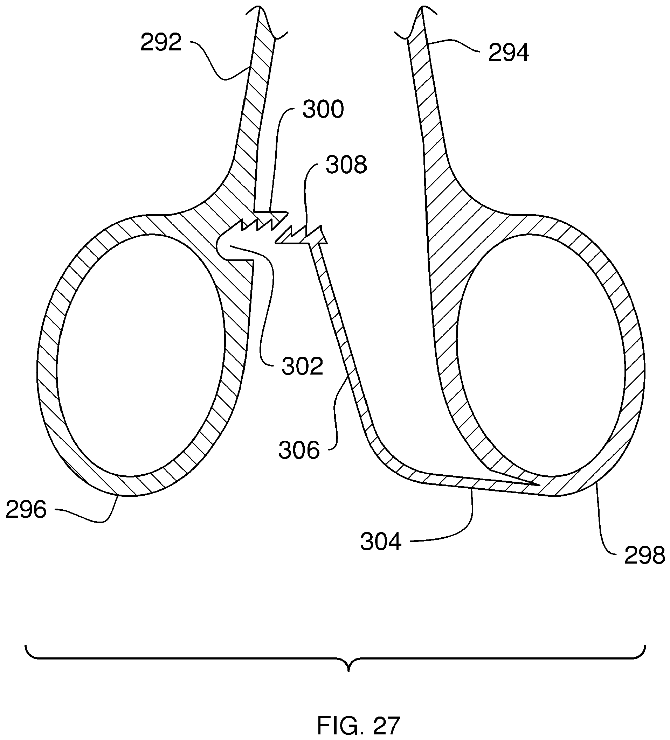

As illustrated in FIG. 27, the latching member **300** of the first finger engaging member **296** extends out from the first finger engaging member toward the second finger engaging member **298.** The notch **302** can have any geometric shape, but preferably a U-shape with the latching member **300** being positioned directly above or below and adjacent to the U-shaped notch. The notch **302** is located so as to be aligned with the ratcheting head **308** of the latching member **304.** The notch **302** is configured to receive the ratcheting head **308** of the latching member **304,** and to allow the ratcheting head **308** to engage and disengage from latching member **300.** The latching member **304** of the second finger engaging member **298** extends out from the bottom of the second finger engaging member toward the first finger engaging member **296.** The arm **306** is attached to the free end of the latching member **304** with an arcuate connection, and the ratcheting head **308** is attached to the free end of the arm **306.** The latching member **304** and the arm **306** are flexible allowing for the free travel of the ratcheting head **308,** with respect to the second finger engaging member **298.** The latching member **304** extends outwardly and upwardly from the interior of the second finger engaging member **298,** while the arm **306** extends outwardly and upwardly from the free end of the latching member **306.**

The ratcheting teeth of the latching member **300** and the ratcheting teeth of the ratcheting head **308** are adapted to join and lock together when engaged by squeezing the finger engaging members **296, 298** together. The teeth are able to disengage when pulled apart by flexing the first and second elongated members **292, 294** when an opposing force is applied to the finger engaging members **296, 298** by pushing with the thumb and pulling with the fingers of the operating hand thereby separating the parallel engaged teeth.

It can be appreciated that the ambidextrous locking clamp system **290** can be used with either the left or right hand with identical methods of separating and disengaging the teeth of the first latching member **300** and the ratcheting head **308.**

The latching members **80, 90, 130, 140, 160, 170, 190, 194, 210, 214, 230, 236, 260, 264, 280, 284, 300, 304** can have indicators thereon and can be adapted to be universally used in many orientations.

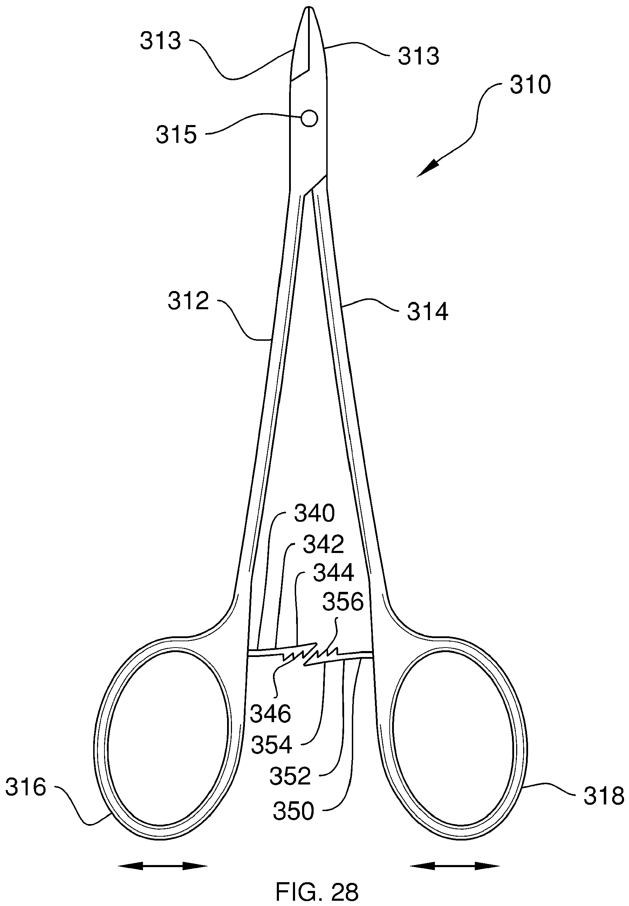

In FIG. 28, a new and improved ambidextrous locking clamp system **310** of the present technology for allowing the use of a hand operated device by a right or left handed user is illustrated and will be described. More particularly, the ambidextrous locking clamp system **310** has a first elongated member **312** and a second elongated member **314** each having a working head **313,** wherein the working heads can be, but not limited to, a gripping jaw or a cutting edge. The second elongated member **314** is connected to the first elongated member **312** via a hinge **315.** The first elongated member **312** has a first finger engaging member **316** located opposite the working head **313** with the hinge **315** located between the working head **313** and the first finger engaging member **316,** and a first latching member **340** extending away from the first elongate member **312** or the first finger engaging member **316.** The second elongated member **314** has a second finger engaging member **318** located opposite the working head **313** with the hinge **315** located between the working head **313** and the second finger engaging member **318,** and a second latching member **350** extending away from the second elongate member **314** or the second finger engaging member **318.** The first and second latching members **340, 350** are oriented toward each other so as to releasably engage with each other. The first and second elongated members **312, 314** can be made from any suitable material having reflex memory.

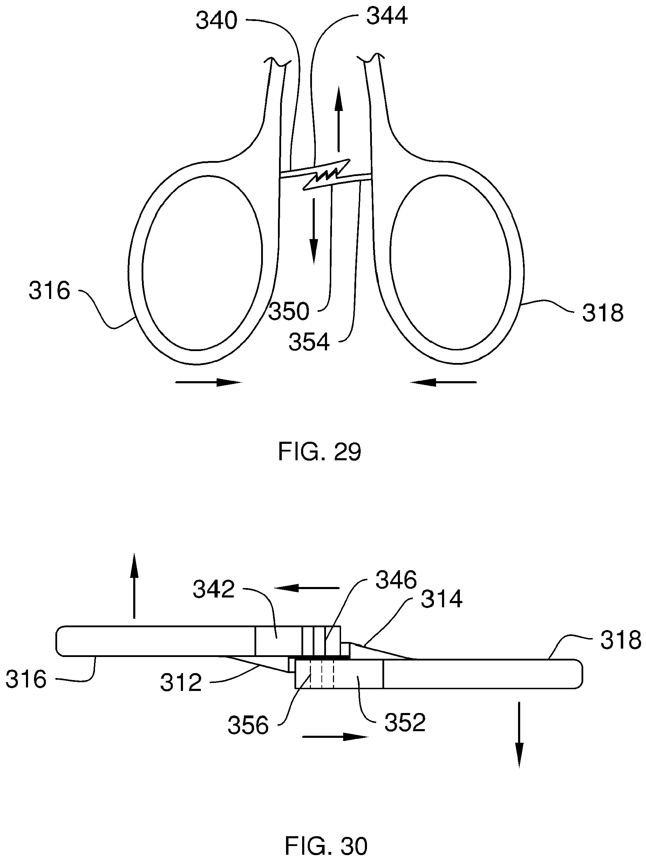

The first and second latching members **340, 350** each have a flexible arm **342, 352,** a ratcheting head **344, 354** located at a free end of the flexible arm **342, 352** respectively. The first ratcheting head **344** has a plurality of ratcheting teeth **346** oriented toward or away from the working head **313.** The second ratcheting head **354** has a plurality of ratcheting teeth **356** oriented in a direction opposite the first ratcheting head **344** to join and lock together when engaged by squeezing the finger engaging members **316, 318** together, as best illustrated in FIG. 29.

It can be appreciated that to operate the working heads **313** a right or left handed user would insert a thumb in either the first or second finger engaging member **316, 318,** and at least one finger in the free finger engaging member opposite the one receiving the thumb. The user would then provide an engaging motion until the ratcheting teeth **346, 356** overlap one another in succession until desire tension or working head force is achieved. The engaging motion is produced by moving the finger engaging members **316, 318** of the first and second members **312, 314** toward each other so as to flex the flexible arms **342, 352** away from each other, thereby interlocking the ratcheting teeth **346, 356** together and locking the ambidextrous locking clamp system **310.**

As best illustrated in FIG. 30, to disengage the ratcheting teeth **346, 356** and release tension or working head force, the user would produce a disengaging motion that is perpendicular to the engaging motion until the ratcheting teeth are slidably disengaged from each other. The disengaging motion is produced by flexing the first and second elongated members **312, 314** in opposite directions by applying an opposing force to the first and second finger engaging members **316, 318** by pushing with the thumb of the operating hand of the user on one of the finger engaging members and pulling with the inserted finger on the other finger engaging member thereby slidably separating the ratcheting teeth. The first and second finger engaging members **316, 318** can then be pulled apart to unlock the ambidextrous locking clamp system **310** or re-engage the ratcheting teeth in a different position to change the tension.

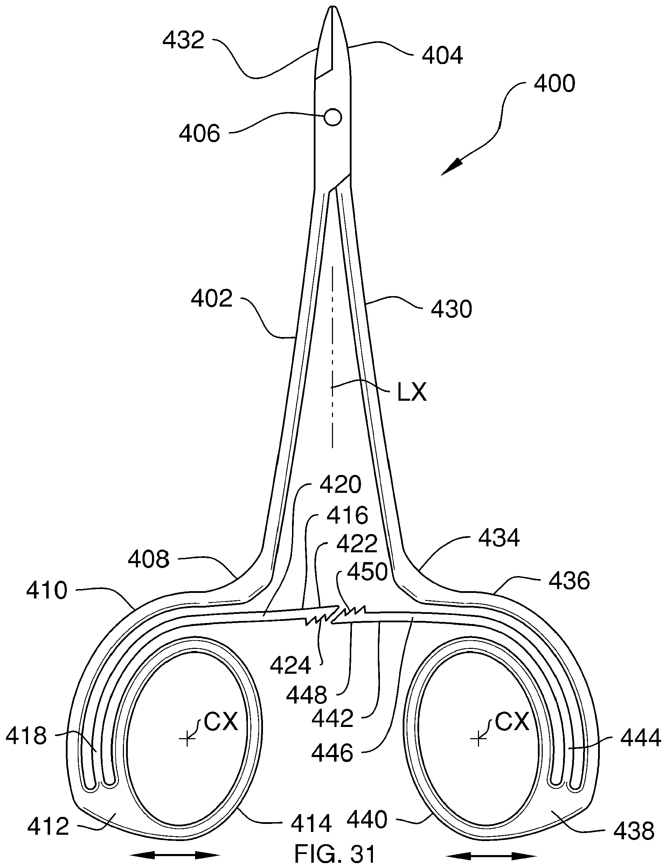

In FIG. 31, a new and improved ambidextrous locking clamp system **400** of the present technology for allowing the use of a hand operated device by a right or left handed user is illustrated and will be described. More particularly, the ambidextrous locking clamp system **400** has a first elongated member **402** and a second elongated member **430** each having a working head **404, 432,** respectively, wherein the working heads can be, but not limited to, a gripping jaw or a cutting edge. The second elongated member **430** is connected to the first elongated member **402** via a hinge **406.** The first and second elongated members **402, 430** each have a corresponding finger engaging member **414, 440** located opposite of their respective working heads **404, 432.** When assembled, the ambidextrous locking clamp system **400** defines a longitudinal axis **LX** that passes through the hinge **406.**

The first elongated member **402** includes a first finger engaging member arm **410,** a first finger engaging member **414,** and a first latching member **416.** The first finger engaging member arm **410** transitions from an end of the first elongated member **402** byway of a first member transitioning section **408.** The first member transitioning section **408** is thicker than the first elongated member **402** and first finger engaging member arm **410.** The first member transitioning section **408** extends away from the longitudinal axis **LX** to be exterior of the first elongated member **402.**

The first finger engaging member arm **410** has an arcuate or curved profile that extends from the first member transitioning section **408** in a direction away from the longitudinal axis **LX** to be exterior of the first member transitioning section **408** and opposite the working head **404.**

The first finger engaging member **414** is located opposite the working head **404** with the hinge **406** located between the working head **404** and the first finger engaging member **414.** The first finger engaging member **414** transitions from an end of the first finger engaging member arm **410** by way of a first member finger transitioning section **412.** The first member finger transitioning section **412** is thicker than the first finger engaging member arm **410** and the first finger engaging member **414.** The first member finger transitioning section **412** extends toward the longitudinal axis **LX** to be interior of the first finger engaging member arm **410.** Consequently, the first finger engaging member **414** is interior of the first finger engaging member arm **410** toward the longitudinal axis **LX.**

The first finger engaging member **414** defines a substantially circular or oval opening having a central axis **CX** and a configuration capable of receiving at least one finger or digit of a user. The first finger engaging member **414** has a portion thereof in a spaced or offset relationship from the first finger engaging member arm **410** to define a space therebetween. The portion of the first finger engaging member **414** that is offset from the first finger engaging member arm **410** has an arcuate or curved profile that corresponds with that of the first finger engaging member arm **410.**

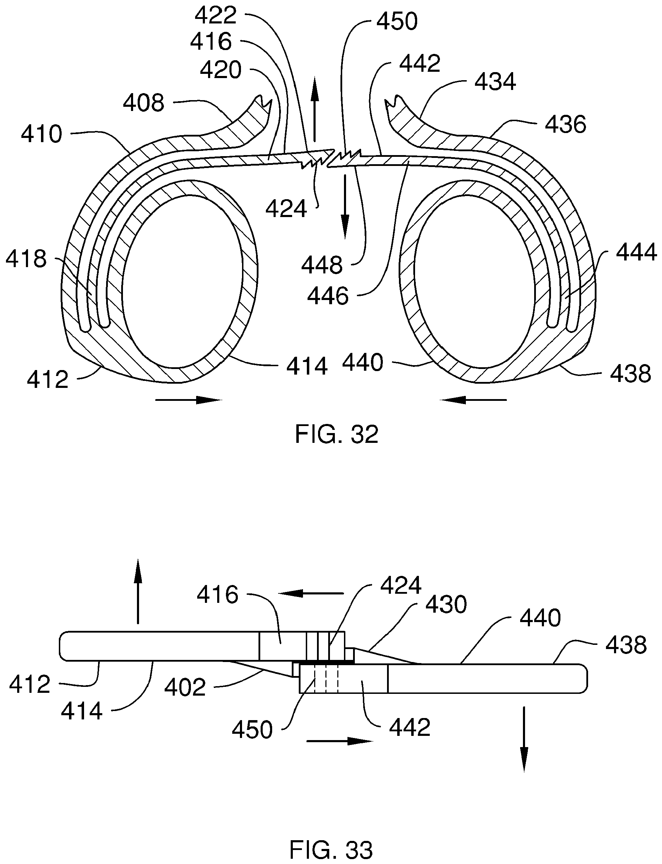

As illustrated in FIG. 32, the first latching member **416** has a first arm section **418,** a second arm section **420,** and a ratcheting head **422.** The first arm section **418** extends away from the first member finger transitioning section **412** in the space between the first finger engaging member arm **410** and the first finger engaging member **414.** The first arm section **418** has an arcuate or curved profile that corresponds with that of the first finger engaging member arm **410** and the portion of the first finger engaging member **414** that is offset from the first finger engaging member arm **410.** Consequently, the first arm section **418** defines a gap between the first finger engaging member arm **410** and the portion of the first finger engaging member **414** that is offset from the first finger engaging member arm **410.**

The second arm section **420** extends away from the first arm section **418** toward the longitudinal axis **LX.** The ratcheting head **422** is located at a free end of the second arm section **420.** The ratcheting head **422** features ratcheting teeth **424** extending in a direction aligned with the longitudinal axis **LX.**

The second elongated member **430** is similar in structure to and a mirror of the first elongated member **402.** The second elongated member **430** includes a second finger engaging member arm **436,** a second finger engaging member **440,** and a second latching member **442.** The second finger engaging member arm **436** transitions from an end of the second elongated member **430** by way of a second member transitioning section **434.** The second member transitioning section **434** is thicker than the second elongated member **430** and the second finger engaging member arm **436.** The second member transitioning section **434** extends away from the longitudinal axis **LX** to be exterior of the second elongated member **430.**

The second finger engaging member arm **436** has an arcuate or curved profile that extends from the second member transitioning section **434** in a direction away from the longitudinal axis **LX** to be exterior of the second member transitioning section **434** and opposite the working head **432.**

The second finger engaging member **440** is located opposite the working head **432** with the hinge **406** located between the working head **432** and the second finger engaging member **440.** The second finger engaging member **440** transitions from an end of the second finger engaging member arm **436** by way of a second member finger transitioning section **438.** The second member finger transitioning section **438** is thicker than the second finger engaging member arm **436** and the second finger engaging member **440.** The second member finger transitioning section **438** extends toward the longitudinal axis **LX** to be interior of the second finger engaging member arm **436.** Consequently, the second finger engaging member **440** is interior of the second finger engaging member arm **436** toward the longitudinal axis **LX.**

The second finger engaging member **440** defines a substantially circular or oval opening having a central axis **CX** and a configuration capable of receiving at least one finger or digit of the user. The second finger engaging member **440** has a portion thereof in a spaced or offset relationship from the second finger engaging member arm **436** to define a space therebetween. The portion of the second finger engaging member **440** that is offset from the second finger engaging member arm **436** has an arcuate or curved profile that corresponds with that of the second finger engaging member arm **436.**

As illustrated in FIG. 32, the second latching member **442** has a first arm section **444,** a second arm section **446,** and a ratcheting head **448.** The first arm section **444** extends away from the second member finger transitioning section **438** in the space between the second finger engaging member arm **436** and the second finger engaging member **440.** The first arm section **444** has an arcuate or curved profile that corresponds with that of the second finger engaging member arm **436** and the portion of the second finger engaging member **440** that is offset from the second finger engaging member arm **436.** Consequently, the first arm section **444** defines a gap between the second finger engaging member arm **436** and the portion of the second finger engaging member **440** that is offset from the second finger engaging member arm **436.**

The second arm section **446** extends away from the first arm section **444** toward the longitudinal axis **LX.** The ratcheting head **448** is located at a free end of the second arm section **446.** The ratcheting head **448** features ratcheting teeth **450** extending in a direction aligned with the longitudinal axis **LX.**

The first and second elongated members **402, 430,** the first and second finger engaging member arms **410, 436,** the first arm sections **418, 444** of the first and second latching members **416, 442** and/or the second arm sections **420, 446** of the first and second latching members **416, 442** can be made from any suitable material having reflex memory.

As illustrated in FIG. 33, the ratcheting teeth **424, 450** of the first and second latching members **416, 442** are adapted to join and lock together when engaged by squeezing the first and second finger engaging members **414, 440** together. It can be appreciated that to operate the working heads **404, 432** a right or left handed user would insert a thumb in either the first or second finger engaging member **414, 440,** and insert at least one finger in the free finger engaging member opposite the one receiving the thumb. The user would then provide an engaging motion until the ratcheting teeth **424, 450** overlap one another in succession until desire tension or working head force is achieved. The engaging motion is produced by moving the first and second finger engaging members **414, 440** of the first and second elongated members **402, 430** toward each other so that the ratcheting heads **422, 448** move toward each other, thereby interlocking the ratcheting teeth **424, 450** together and locking the ambidextrous locking clamp system **400.**

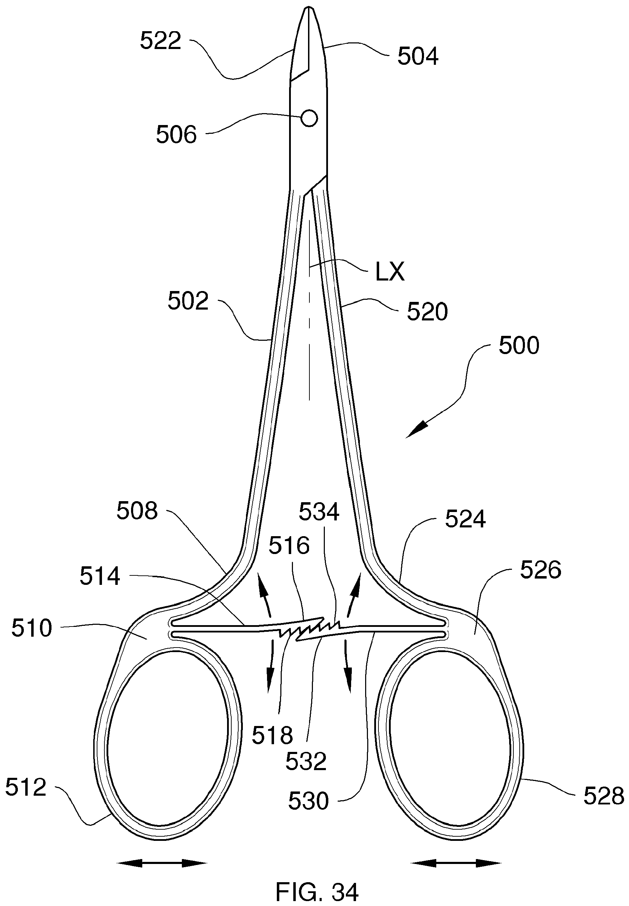

Referring to FIG. 34, the present technology can include a new and improved ambidextrous locking clamp system **500** for allowing the use of a hand operated device by a right or left handed user. More particularly, the ambidextrous locking clamp system **500** has a first elongated member **502** and a second elongated member **520** each having a working head **504, 522,** respectively, wherein the working heads can be, but not limited to, a gripping jaw or a cutting edge. The first and second elongated members **502, 520** can be rotatably connected to each other via a hinge **506.** The first and second elongated members **502, 520** each have a corresponding finger engaging member **512, 528** located opposite of their respective working heads **504, 522.** When assembled, the ambidextrous locking clamp system **500** defines a longitudinal axis **LX** that passes through the hinge **506.**

The first elongated member **502** includes a first transitioning section **508,** a first shoulder section **510,** and a first latching member **514.** The first transitioning section **508** can be curve outwardly and away from the first elongated member **502.** The first shoulder section **510** transitions from an end of the first transitioning section **508.**

The first finger engaging member **512** is located opposite the working head **504** with the hinge **506** located between the working head **504** and the first finger engaging member **512.** The first finger engaging member **512** transitions from a side of the first shoulder section **510** opposite the first transitioning section **508.** A section of the first finger engaging member **512** adjacent to its connection or transition from the first shoulder section **510** is spaced apart from the first transitioning section **508** to define a space between a convex or outer side of the curved first transitioning section **508** and a convex or outer side of a section of the curved first finger engaging member **512.** The first finger engaging member **512** defines a substantially circular or oval opening having a configuration capable of receiving at least one finger or digit of a user.

The first latching member **514** can include a first arm extending away from the first shoulder section **510** in the space between the first transitioning section **508** and the first finger engaging member **512.**

The first latching member **514** extends toward the longitudinal axis **LX** when the locking clamp system **500** is in a closed configuration. A ratcheting head **516** is located at a free end of the first latching member **514.** The ratcheting head **516** can feature a tapered or pointed free end, and includes ratcheting teeth **518** extending in a direction aligned with the longitudinal axis **LX** when the locking clamp system **500** is in the closed configuration. A lateral width or profile of the ratcheting teeth **518** is substantially perpendicular to the longitudinal axis **LX** when the locking clamp system **500** is in the closed configuration.

The second elongated member **520** is similar in structure to and a mirror of the first elongated member **502,** except for an orientation of the ratcheting teeth. The second elongated member **520** includes a second transitioning section **524,** a second shoulder section **526,** and a second latching member **530.** The second transitioning section **524** can be curve outwardly and away from the second elongated member **520.** The second shoulder section **526** transitions from an end of the second transitioning section **524.**

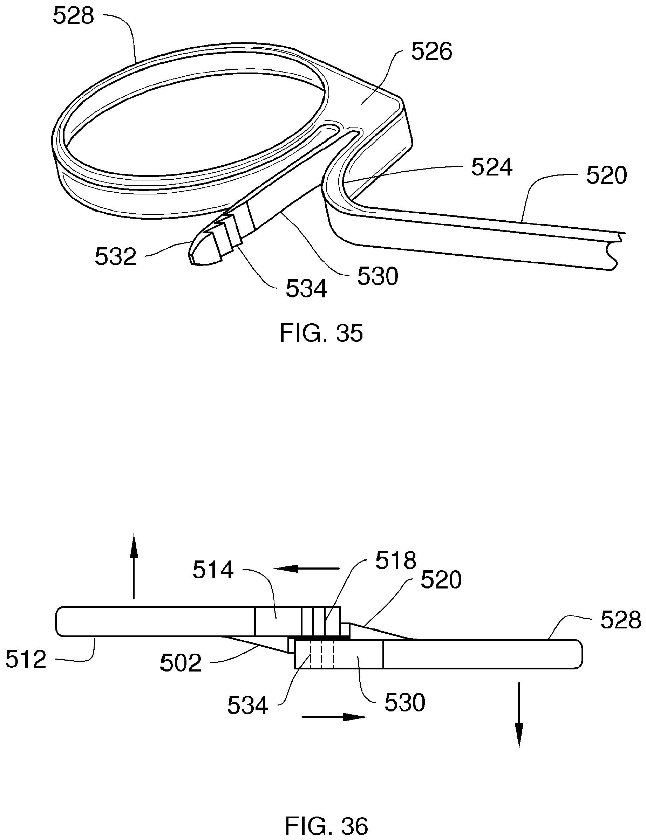

As best illustrated in FIG. 35, the second finger engaging member **528** is located opposite the working head **522** with the hinge **506** located between the working head **522** and the second finger engaging member **528.** The second finger engaging member **528** transitions from a side of the second shoulder section **526** opposite the second transitioning section **524.** A section of the second finger engaging member **528** adjacent to its connection or transition from the second shoulder section **526** is spaced apart from the second transitioning section **524** to define a space between a convex or outer side of the curved second transitioning section **524** and a convex or outer side of a section of the curved second finger engaging member **528.** The second finger engaging member **528** defines a substantially circular or oval opening having a configuration capable of receiving at least one finger or digit of a user.

The second latching member **530** extends toward the longitudinal axis **LX** when the locking clamp system **500** is in a closed configuration. A ratcheting head **532** is located at a free end of the second latching member **530.** The ratcheting head **532** can feature a tapered or pointed free end, and includes ratcheting teeth **534** extending in a direction aligned with the longitudinal axis **LX** when the locking clamp system **500** is in the closed configuration. A lateral width or profile of the ratcheting teeth **534** is substantially perpendicular to the longitudinal axis **LX** and facing the ratcheting teeth **548** of the first latching member **514** when the locking clamp system **500** is in the closed configuration.

The first and second transitioning sections **508, 524,** and/or the first and second latching members **514, 530** can be made from any suitable material having reflex memory.

As illustrated in FIG. 34, the ratcheting teeth **518, 534** of the first and second latching members **514, 530** are adapted to join and lock together when engaged by squeezing the first and second finger engaging members **512, 528** together. It can be appreciated that to operate the working heads **504, 522** a right or left handed user would insert a thumb in either the first or second finger engaging member **512, 528,** and insert at least one finger in the free finger engaging member opposite the one receiving the thumb. The user would then provide an engaging motion until the ratcheting teeth **518, 538** begin to flex apart and overlap one another in succession until desire tension or working head force is achieved. The engaging motion is produced by moving the first and second finger engaging members **512, 528** of the first and second elongated members **502, 520** toward each other so that the ratcheting heads **516, 532** move toward each other, thereby interlocking the ratcheting teeth **518, 534** together and locking the ambidextrous locking clamp system **500.**

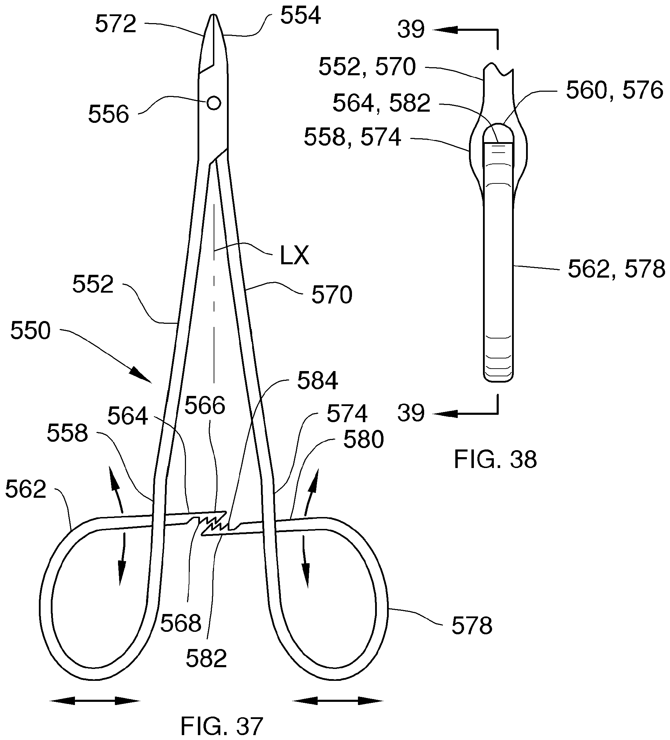

Referring to FIG. 37, the present technology can include a new and improved ambidextrous locking clamp system **550** for allowing the use of a hand operated device by a right or left handed user. More particularly, the ambidextrous locking clamp system **550** has a first elongated member **552** and a second elongated member **570** each having a working head **554, 572,** respectively, wherein the working heads can be, but not limited to, a gripping jaw or a cutting edge. The first and second elongated members **552, 570** can be rotatably connected to each other via a hinge **556.** The first and second elongated members **552, 570** each have a corresponding finger engaging member **562, 578** located opposite of their respective working heads **554, 572.** When assembled, the ambidextrous locking clamp system **550** defines a longitudinal axis **LX** that passes through the hinge **556.**

The first elongated member **552** includes a first receipt section **558** that defines a first opening or bore **560** therethrough, as best illustrated in FIG. 38. The first receipt section **558** can have a width greater than the first elongated member **552,** thereby creating a widened section to accommodate the first bore **560.**

The first finger engaging member **562** is located opposite the working head **554** with the hinge **556** located between the working head **554** and the first finger engaging member **562.** The first finger engaging member **562** transitions from the first receipt section **558,** and continue in a substantially circular or oval path having a configuration capable of receiving at least one finger or digit of a user.

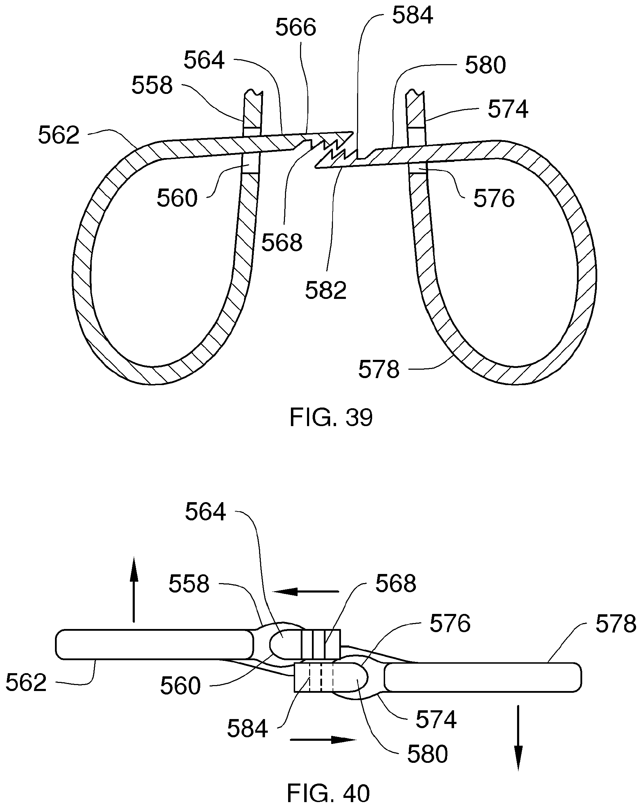

The first finger engaging member **562** includes a first latching member **564** extending or transitioning therefrom, which is received or inserted through the first bore **560,** as best illustrated in FIGS. 38 and 39. The first latching member **564** extends toward the longitudinal axis **LX** when the locking clamp system **550** is in a closed configuration. A ratcheting head **566** is located at a free end of the first latching member **564,** which includes ratcheting teeth **568.** A lateral width or profile of the ratcheting teeth **568** is substantially perpendicular to the longitudinal axis **LX** when the locking clamp system **550** is in the closed configuration.

The second elongated member **570** is similar in structure to and a mirror of the first elongated member **552,** except for an orientation of the ratcheting teeth. The second elongated member **570** includes a second receipt section **574** that defines a second opening or bore **576** therethrough, as best illustrated in FIG. 38. The second receipt section **574** can have a width greater than the second elongated member **570,** thereby creating a widened section to accommodate the second bore **576.**

The second finger engaging member **578** is located opposite the working head **572** with the hinge **556** located between the working head **572** and the second finger engaging member **578.** The second finger engaging member **578** transitions from the second receipt section **574,** and continue in a substantially circular or oval path having a configuration capable of receiving at least one finger or digit of a user.

The second finger engaging member **578** includes a second latching member **580** extending or transitioning therefrom, which is received or inserted through the second bore **576,** as best illustrated in FIGS. 38 and 39. The second latching member **580** extends toward the longitudinal axis **LX** when the locking clamp system **550** is in a closed configuration. A ratcheting head **582** is located at a free end of the second latching member **580,** which includes ratcheting teeth **584.** A lateral width or profile of the ratcheting teeth **584** is substantially perpendicular to the longitudinal axis **LX** and facing the ratcheting teeth **568** of the first latching member **564** when the locking clamp system **550** is in the closed configuration.

The first and second finger engaging members **562, 578,** and/or the first and second latching members **564, 580** can be made from any suitable material having reflex memory. It can be appreciated that the first and second finger elongated members **552, 570,** the first and second finger engaging members **562, 578,** and the first and second latching members **564, 580** can each be a single integral member that is bent to form the first and second finger engaging members **562, 578,** and with their corresponding latching member inserted through their corresponding bore **560, 576,** respectively.

As illustrated in FIG. 40, the ratcheting teeth **568, 584** of the first and second latching members **564, 580** are adapted to join and lock together when engaged by squeezing the first and second finger engaging members **562, 578** together. It can be appreciated that to operate the working heads **554, 572** a right or left handed user would insert a thumb in either the first or second finger engaging member **562, 578,** and insert at least one finger in the free finger engaging member opposite the one receiving the thumb. The user would then provide an engaging motion until the ratcheting teeth **568, 584** begin to flex apart and overlap one another in succession until desire tension or working head force is achieved. The engaging motion is produced by moving the first and second finger engaging members **562, 578** of the first and second elongated members **552, 570** toward each other so that the ratcheting heads **566, 582** move toward each other, thereby interlocking the ratcheting teeth **568, 584** together and locking the ambidextrous locking clamp system **550.**

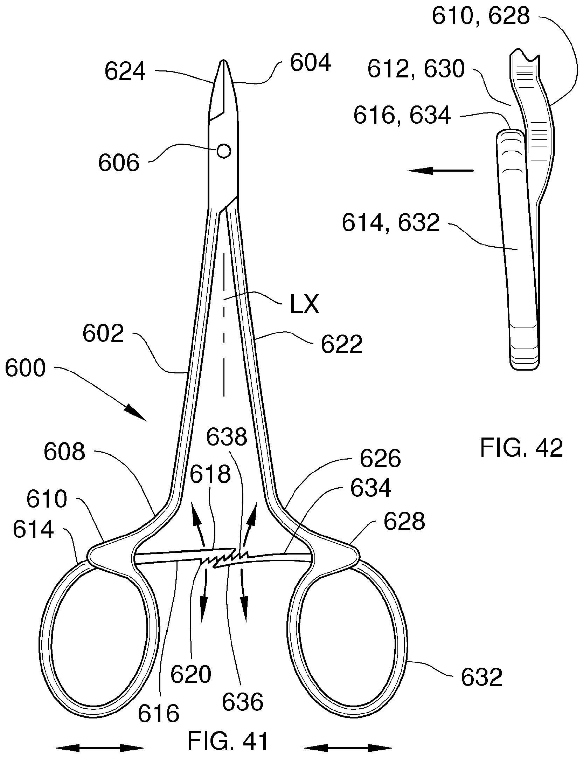

Referring to FIG. 41, the present technology can include a new and improved ambidextrous locking clamp system **600** for allowing the use of a hand operated device by a right or left handed user. More particularly, the ambidextrous locking clamp system **600** has a first elongated member **602** and a second elongated member **622** each having a working head **604, 624,** respectively, wherein the working heads can be, but not limited to, a gripping jaw or a cutting edge. The first and second elongated members **602, 622** can be rotatably connected to each other via a hinge **606.** The first and second elongated members **602, 622** each have a corresponding finger engaging member **614, 632** located opposite of their respective working heads **604, 624.** When assembled, the ambidextrous locking clamp system **600** defines a longitudinal axis **LX** that passes through the hinge **606.**

The first elongated member **602** includes a first transitioning section **608,** a first shoulder section **610,** and a first latching member **616.** The first transitioning section **608** can be curve outwardly and away from the first elongated member **602.** The first shoulder section **610** transitions from an end of the first transitioning section **608,** and can be bent or configured to define a first open space, notch or recess **612,** as best illustrated in FIG. 42. The first shoulder section **610** can have a width greater than that of the first elongated member **602** and/or the first transitioning section **608,** thereby creating a widened section.

The first finger engaging member **614** is located opposite the working head **604** with the hinge **606** located between the working head **604** and the first finger engaging member **614.** The first finger engaging member **614** transitions from a side of the first shoulder section **610** opposite the first transitioning section **608.** The first finger engaging member **614** defines a substantially circular or oval opening having a configuration capable of receiving at least one finger or digit of a user.

The first finger engaging member **614** includes a first latching member **616** extending or transitioning therefrom, which is received in the first recess **612,** as best illustrated in FIG. 42. The first latching member **616** extends toward the longitudinal axis **LX** when the locking clamp system **600** is in a closed configuration. A ratcheting head **618** is located at a free end of the first latching member **616,** which includes ratcheting teeth **620.** A lateral width or profile of the ratcheting teeth **620** is substantially perpendicular to the longitudinal axis **LX** when the locking clamp system **600** is in the closed configuration.

The second elongated member **622** is similar in structure to and a mirror of the first elongated member **602,** except for an orientation of the ratcheting teeth. The second elongated member **622** includes a second transitioning section **626,** a second shoulder section **628,** and a second latching member **634.** The second transitioning section **626** can be curve outwardly and away from the second elongated member **622.** The second shoulder section **628** transitions from an end of the second transitioning section **626,** and can be bent or configured to define a second open space, notch or recess **630,** as best illustrated in FIG. 42. The second shoulder section **628** can have a width greater than that of the second elongated member **622** and/or the second transitioning section **626,** thereby creating a widened section.

The second finger engaging member **632** is located opposite the working head **624** with the hinge **606** located between the working head **624** and the second finger engaging member **632.** The second finger engaging member **632** transitions from a side of the second shoulder section **628** opposite the second transitioning section **626.** The second finger engaging member **632** defines a substantially circular or oval opening having a configuration capable of receiving at least one finger or digit of a user.

The second finger engaging member **632** includes a second latching member **634** extending or transitioning therefrom, which is received in the second recess **630,** as best illustrated in FIG. 42. The second latching member **634** extends toward the longitudinal axis **LX** when the locking clamp system **600** is in a closed configuration. A ratcheting head **636** is located at a free end of the second latching member **634,** which includes ratcheting teeth **638.** A lateral width or profile of the ratcheting teeth **638** is substantially perpendicular to the longitudinal axis **LX** when the locking clamp system **600** is in the closed configuration.

The first and second transitioning sections **608, 626,** the first and second finger engaging members **614, 632** and/or the first and second latching members **616, 634** can be made from any suitable material having reflex memory.

The ratcheting teeth **620, 638** of the first and second latching members **616, 634** are adapted to join and lock together when engaged by squeezing the first and second finger engaging members **614, 632** together. It can be appreciated that to operate the working heads **604, 624** a right or left handed user would insert a thumb in either the first or second finger engaging member **614, 632,** and insert at least one finger in the free finger engaging member opposite the one receiving the thumb. The user would then provide an engaging motion until the ratcheting teeth **620, 638** begin to flex apart and overlap one another in succession until desire tension or working head force is achieved. The engaging motion is produced by moving the first and second finger engaging members **614, 632** of the first and second elongated members **602, 622** toward each other so that the ratcheting heads **618, 636** move toward each other, thereby interlocking the ratcheting teeth **620, 638** together and locking the ambidextrous locking clamp system **600.**

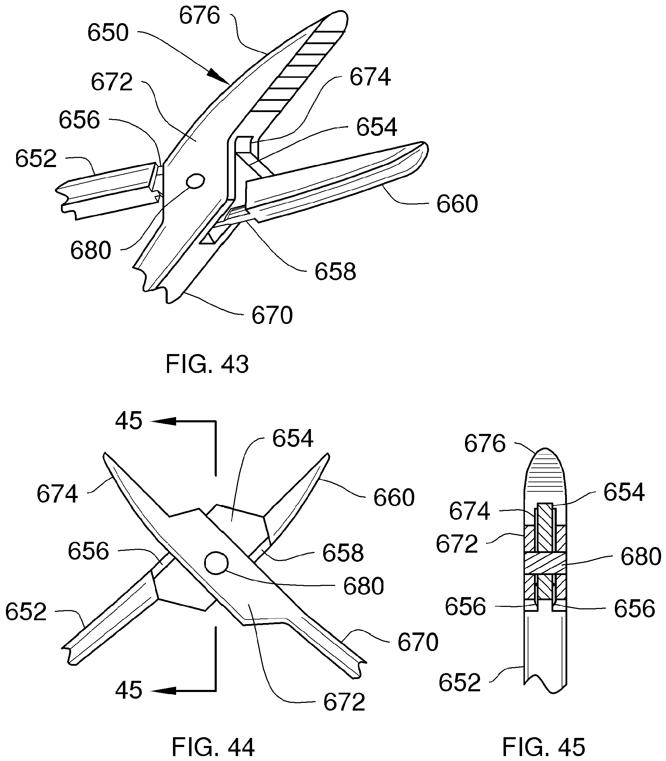

Referring to FIGS. 43-48, any embodiment of the present technology can include a new and improved working head and/or pivoting portion **650** for reducing or eliminating any lateral lag association with the pivoting portion including a hinge **680.** One disadvantage of known types of hand operated locking devices is that the tolerance in the pivoting portion of the working head creates a lateral lag where a male portion goes through a female portion at the pivot point. This lateral lag creates unwanted wiggle or uncontrolled operation. Lateral stiffness at this pivot portion is beneficial to keep the locking fingers or latching members to stay engaged. However, it is not desirable to tighten the tolerance in the pivot portion to remove this lateral lag to the point where it does not function properly to overcome this disadvantage. In the locked position as designed, there is too much space at the pivot point with traditional manufacturing process.

The embodiments of the present technology can utilize a pivot portion **650** associated with a first elongated member **652** and a second elongated member **670** each having a working head **660, 676,** respectively, wherein the working heads can be, but not limited to, a gripping jaw or a cutting edge. The first and second elongated members **652, 670** can be rotatably connected to each other via a hinge **680.** The first and second elongated members **652, 670** each have a corresponding finger engaging member (not shown) located opposite of their respective working heads **660, 676.**

In the exemplary, the first elongated member **652** can include a planar male potion **654** transitioning therefrom, and the working head **660** transitions from the male portion **654** from a side opposite the first elongated member **652.** The hinge **680** passes through or is pivotably associated with generally central location of the male portion **654.** The male portion **654** can be configured so that the first elongated member **652** and the working head **660** are offset from each other orientated on either side of the hinge **680.** The male portion **654** has a thickness less than the first elongated member **652** and the working head **660.** It can be appreciated that the planar male portion can be associated with the second elongated member and the female portion with the first elongated member.

A primary wedge **656** extends from the male portion **654** at a location adjacent to a juncture or transitioning portion of the first elongated member **652** and the male portion **654.** The primary wedge **656** can be two wedges extending out from opposite sides of the male portion **654.** Each primary wedge **656** can taper from an exterior side of the male portion **654** toward the hinge to create a primary wedge thickness.

Optionally, a second wedge **658** can extend from the male portion **654** at a location adjacent to a juncture or transitioning portion of the working head **676** and the male portion **654.** The secondary wedge **658** can be two wedges extending out from opposite sides of the male portion **654.** Each secondary wedge **658** can taper from an exterior side of the male portion **654** toward the hinge to create a secondary wedge thickness.

The second elongated member **670** can include a female potion **672** transitioning therefrom, which defines a slot **674.** The working head **676** transitions from the female portion **672** from a side opposite the second elongated member **670.** The hinge **680** passes through or is pivotably associated with generally central location of the female portion **672.** The female portion **672** can be configured so that the second elongated member **670** and the working head **676** are offset from each other orientated on either side of the hinge **680.**

The slot **674** of the female portion **672** is configured to receive the male portion **654** of the first elongated member **652,** so that male portion **654** is pivotable therein about the hinge **680.** A thickness of the male portion **654** is less than a thickness of the slot **674,** thereby create a gap between both sides of the male portion **654** and the sides of the female portion **672** that defines the slot **674,** as best illustrated in FIG. 45.

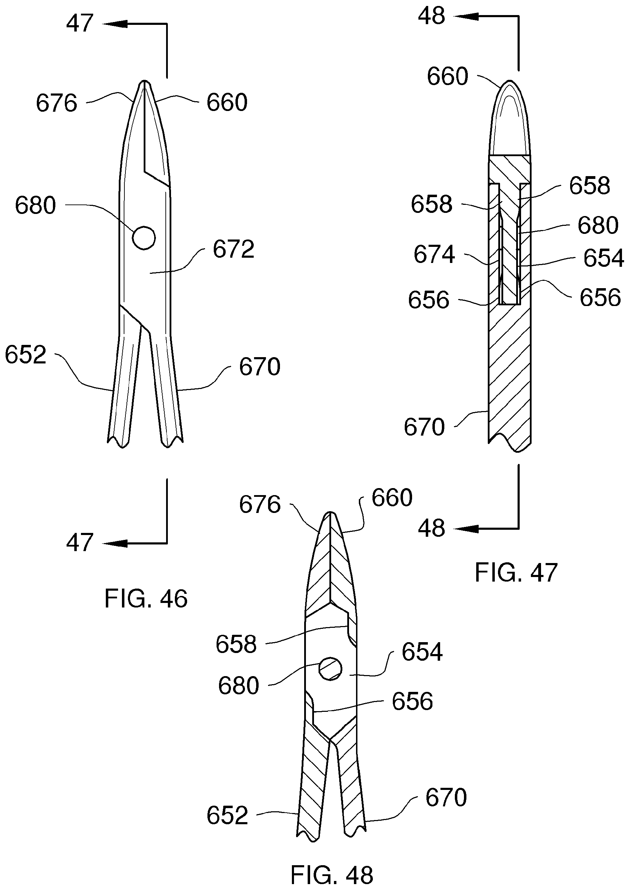

The gap allows free pivotable motion of the first and second elongated members **652, 670** in relation to the hinge **680,** between an open position illustrated in FIG. 44 and a closed position illustrated in FIG. 46. This pivoting movement pivots the working heads **660, 676** together.

The primary and secondary wedges **656, 658** are offset from each other orientated on either side of the hinge **680.** This offset orientation allows the primary and secondary wedges **656, 658** to be removed or withdrawn from the slot **674** when the present technology is in an open position, as best illustrated in FIGS. 43 and 44.

During a closing operation associated with interlocking the ratcheting teeth together and locking the ambidextrous locking clamp system, the primary and secondary wedges **656, 658** are pivoted into the slot **674** so that a free edge of each of the wedges contact the side of the female portion **672** that defines the slot **674,** respectfully. The wedges **656, 658** fill the gap between the male portion **654** and the side of the female portion **672** that defines the slot **674,** thereby eliminating all lateral lag or wiggle associated with operating the present technology tool, as best illustrated in FIG. 47.

It can be appreciated that the ratcheting heads of the embodiments of the present technology can feature a tapered or pointed free end.

The ratcheting heads of the present technology, respectively, can move by:
Pivoting the first and second finger engaging members about the first and second member transitioning sections;
Flexing the first and second finger engaging member arms;
Pivoting the first arm sections about the first and second member finger transitioning sections;
Flexing the first arm sections; and/or
Flexing the second arm sections.

To disengage the ratcheting teeth and release tension of working head force, the user would produce a disengaging motion that is perpendicular to the engaging motion until the ratcheting teeth are slidably disengaged from each other. The disengaging motion may be produced by moving the first and second elongated members in opposite directions by applying an opposing force to the first and second finger engaging members by pushing with the thumb of the operating hand of the user on one of the finger engaging members and pulling with the inserted finger on the other finger engaging member thereby slidably separating the ratcheting teeth. The first and second finger engaging members can then be pulled apart to unlock the ambidextrous locking clamp system or re-engage the ratcheting teeth in a different position to change the tension.

It can be appreciated that the engaging and disengaging motions can be initiated by either a left or right handed user in the same manner by simply inserting the thumb of the operating hand in one of the first and second finger engaging members and the at least one finger of the same operating hand in the other finger engaging member.

The latching members can have indicators thereon and can be adapted to be universally used in many orientations.

The above described engaging and disengaging motion can be used for all embodiments of the present technology, and in use, it can now be understood that either a right hand or left hand user can operate the ambidextrous locking clamp system. As described above, the user would apply opposing force to the finger engaging members pushing with the thumb and pulling with the fingers of the operating hand thereby separating the engaged teeth of the first and second latching members.

While a preferred embodiment of the ambidextrous locking clamp system has been described in detail, it should be apparent that modifications and variations thereto are possible, all of which fall within the true spirit and scope of the technology. With respect to the above description then, it is to be realized that the optimum dimensional relationships for the parts of the technology, to include variations in size, materials, shape, form, function and manner of operation, assembly and use, are deemed readily apparent and obvious to one skilled in the art, and all equivalent relationships to those illustrated in the drawings and described in the specification are intended to be encompassed by the present technology. For example, any suitable sturdy material may be used for the manufacture of the ambidextrous locking clamp system, such as but not limited to, steel, aluminum, plastics, and composites. And although manipulating objects with a tool having latching members have been described, it should be appreciated that the ambidextrous locking clamp system herein described is also suitable for all types of hand operated locking tools having a at least two hingedly connected arms.

Therefore, the foregoing is considered as illustrative only of the principles of the technology. Further, since numerous modifications and changes will readily occur to those skilled in the art, it is not desired to limit the technology to the exact construction and operation shown and described, and accordingly, all suitable modifications and equivalents may be resorted to, falling within the scope of the technology.

## Claims

1. An ambidextrous locking clamp system (10, 60, 100, 150, 180, 200, 220, 250, 270, 290, 310, 400, 500, 550, 600) for allowing a right hand or left hand user to operate said clamp, said system (10, 60, 100, 150, 180, 200, 220, 250, 270, 290, 310, 400, 500, 550, 600) comprising:
at least one first member comprising:
at least one first elongated body (12) including a planar portion, and at least one first working head (13, 63, 103, 153, 183,203,223,253,273,293,313,404,432, 504, 522, 554, 572, 604, 624, 660, 676) located at an end of said first elongated body (12);
at least one first finger engaging member (16, 66, 156, 186,206,226,256,272,296, 316, 414, 512, 562, 614); and
at least one first latching member (40, 80, 130, 340, 416, 514, 564, 616) including first member ratcheting teeth;
at least one second member comprising:
at least one second elongated body (14) including a female portion (672) defining a slot (674), and at least one second working head (13, 63, 103, 153, 183, 203, 223, 253, 273, 293, 313, 404, 432, 504, 522, 554, 572, 604, 624, 660, 676) located at an end of said second elongated body (14), said slot being configured to receive said planar portion of said first member;
at least one second finger engaging member (18, 68, 158, 188, 208, 228, 258, 278, 298, 318, 440, 528, 578, 632); and
at least one second latching member (50, 90, 140, 350, 442, 530, 580, 634) including second member ratcheting teeth;
wherein said first (12) and second (14) members being in a pivotable relationship about a pivot point so that said first and second working heads (13, 63, 103, 153, 183, 203, 223, 253, 273, 293, 313, 404, 432, 504, 522, 554, 572, 604, 624, 660, 676) move toward each other when said first and second finger engaging member (16, 18; 66, 68; 156, 158; 186, 188; 206, 208; 226, 228; 256, 258; 272, 278; 296, 298; 316, 318; 414, 440; 512, 528; 562, 578; 614, 632) are moved toward each other; and
wherein said first and second member ratcheting teeth being orientated to be engageable with each other by an engaging motion when said first and second finger engaging members (16, 18; 66,68; 156, 158; 186, 188; 206,208; 226,228; 256,258; 272,278; 296, 298; 316, 318; 414, 440; 512, 528; 562, 578; 614, 632) are moved toward each other.

2. The system (10, 60, 100, 150, 180,200,220,250,270,290,310,400, 500, 550, 600) of claim 1, wherein said planar portion includes at least one wedge (656, 658) configured to contact a side of said female portion (672) that defines said slot when said first and second working heads (13, 63, 103, 153, 183, 203, 223, 253, 273, 293, 313, 404, 432, 504, 522, 554, 572, 604, 624, 660, 676) are in a closed position.

3. The system (10, 60, 100, 150, 180, 200, 220, 250, 270, 290, 310, 400, 500, 550, 600)of claim 2, wherein said wedge (656) extends from said planar portion so that an exterior edge of said wedge (656) is flush with an edge of said planar portion.

4. The system (10, 60, 100, 150, 180,200,220,250,270,290,310,400, 500, 550, 600) of claim 2, wherein said wedge (656) includes an exterior side and an interior tapered side, said wedge (656) is configured so that said interior tapered side enters said slot (674) prior to said exterior side during the engaging motion.

5. The system (10, 60, 100, 150, 180,200,220,250,270,290,310,400, 500, 550, 600) of claim 2, wherein said wedge is a pair of wedges (656) each extending from opposite sides of said planar portion away from each other.

6. The system (10, 60, 100, 150, 180, 200, 220, 250, 270, 290, 310, 400, 500, 550, 600) of claim 2, wherein said wedge (656) is adjacent said first elongated body, with said pivot point being located between said wedge (656) and said working head (13, 63, 103, 153, 183, 203, 223, 253, 273, 293, 313, 404, 432, 504, 522, 554, 572, 604, 624, 660, 676) of said first member.

7. The system (10, 60, 100, 150, 180, 200, 220, 250, 270, 290, 310, 400, 500, 550, 600) of claim 2, wherein said planar portion includes at least one secondary wedge (658) configured to contact the side of said female portion (672) that defines said slot (674) when said first and second working heads (13, 63, 103, 153, 183, 203, 223, 253, 273, 293, 313, 404, 432, 504, 522, 554, 572, 604, 624, 660, 676) are in a closed position.

8. The system (10, 60, 100, 150, 180,200,220,250,270,290,310,400, 500, 550, 600) of claim 7, wherein said secondary wedge (658) includes an exterior side and an interior tapered side, said secondary wedge (658) is configured so that said interior tapered side of said secondary wedge (658) enters said slot (674) prior to said exterior side of said secondary wedge (658) during the engaging motion.

9. The system (10, 60, 100, 150, 180, 200, 220, 250, 270, 290, 310, 400, 500, 550, 600) of claim 7, wherein said secondary wedge (658) is a pair of secondary wedges (658) each extending from opposite sides of said planar portion away from each other.

10. The system (10, 60, 100, 150, 180, 200, 220, 250, 270, 290, 310, 400, 500, 550, 600) of claim 7, wherein said secondary wedge (658) is adjacent said working head (13, 63,103,153,183, 203, 223, 253, 273, 293, 313, 404, 432, 504, 522, 554, 572, 604, 624, 660, 676) of said first member, with said pivot point being located between said wedge (656) and said secondary wedge (658).

11. The system (10, 60, 100, 150, 180,200,220,250,270,290,310,400, 500, 550, 600) of claim 7, wherein said wedge (656) and said secondary wedge (658) are located on opposite sides of a longitudinal axis of said first member.

12. The system (10, 60, 100, 150, 180, 200, 220, 250, 270, 290, 310, 400, 500, 550, 600) of claim 1, wherein said first latching member (80, 130, 210, 260, 300, 340, 416, 514, 564) is received through a first member opening (46, 56, 560, 576) defined through said first elongated member (12, 62, 102, 152, 552, 602, 652), and said second latching member (50, 90, 140, 350, 442, 530, 580, 634) is received through a second member opening defined through said second elongated member.

13. The system (10, 60, 100, 150, 180, 200, 220, 250, 270, 290, 310, 400, 500, 550, 600) of claim 12, wherein said first member opening (46,56,560,576) is a recess (612) defined by a bent portion of said first elongated body, and said second member opening is a recess (630) defined by a bent portion of said second elongated body.

14. The system (10, 60, 100, 150, 180, 200, 220, 250, 270, 290, 310, 400, 500, 550, 600) of claim 12, wherein said first member opening (46, 56, 560, 576) is a bore defined through said first elongated body, and said second member opening is a bore (560, 576) defined through said second elongated body.

15. The system (10, 60, 100, 150, 180, 200, 220, 250, 270, 290, 310, 400, 500, 550, 600) of claim 1, wherein said first and second latching members (40, 50; 80, 90; 130, 140; 340,350; 416, 442; 514, 530; 564, 580; 616, 634) each include a free end featuring side edges that tapered toward each other.

16. The system (10, 60, 100, 150, 180,200,220,250,270,290,310,400, 500, 550, 600) of claim 1, wherein said first and second member ratcheting teeth having a configuration for disengaging with each other by sliding said first and second member ratcheting teeth apart by a disengaging motion perpendicular to said engaging motion resulting in moving said first and second latching members (40, 50; 80, 90; 130,140; 340,350; 416,442; 514,530; 564,580; 616,634) away from each other when an opposing force is applied to said first and second finger engaging (156, 158; 186, 188; 206, 208; 226, 228; 256, 258; 276, 278; 296, 298; 316, 318; 410, 436; 414, 440; 512, 528; 562, 578; 614, 632) members.

17. A method of using an ambidextrous locking clamp system (10), said method comprising the steps of:
a) operating a first finger engaging member (16, 66, 156, 186, 206, 226, 256, 272, 296, 316, 414, 512, 562, 614) of a first member and a second finger engaging member (18, 68, 158, 188, 208, 228, 258, 278, 298, 318, 440, 528, 578, 632) of a second member by a user to move said first and second finger engaging members (16, 18; 66, 68; 156, 158; 186, 188; 206, 208; 226, 228; 256, 258; 272, 278; 296, 298; 316, 318; 414, 440; 512, 528; 562, 578; 614, 632) toward each other about a pivot point in an engaging motion, said pivot point being configured to pivotably connect said first and second members to each other;
b) rotating a planar portion of said first member and a female portion (672) of said second member about said pivot point during the engaging motion, said planar portion being receivable in a slot (674) defined in said female portion (672);
c) engaging ratcheting teeth of first and second members with each other by the engaging motion until said ratcheting teeth of said first and second members overlap one another in succession to a user desired tension when a working head (13, 63, 103, 153, 183, 203, 223, 253, 273, 293, 313, 404, 432, 504, 522, 554, 572, 604, 624, 660, 676) of said first member and said second member are in a closed position; and
d) disengaging said ratcheting teeth of said first and second members by a disengaging motion perpendicular to said engaging motion until said ratcheting teeth are slidably disengaged, the disengaging motion being produced by moving said first and second members in opposite directions when an opposing force is applied to said first and second finger engaging members (16, 18; 66, 68; 156, 158; 186, 188; 206, 208; 226, 228; 256,258; 272,278; 296,298; 316, 318; 414,440; 512, 528; 562,578; 614,632) by pushing on at least one of said first and second finger engaging members (16, 18; 66, 68; 156, 158; 186, 188;, 206, 208; 226, 228; 256, 258; 272, 278;, 296, 298; 316, 318; 414, 440; 512, 528; 562, 578; 614, 632) and pulling on the other of said first and second finger engaging members (16, 18; 66, 68; 156, 158; 186, 188; 206, 208; 226, 228; 256,258; 272,278; 296,298; 316, 318; 414,440; 512, 528; 562,578; 614,632) thereby slidably separating said ratcheting teeth of said first and second members out of engagement.

18. The method of claim 17, contacting a wedge extending from said planar portion with a side of said female portion (572) that defines said slot when in the closed position.
